(19) Europäisches Patentamt
European Patent Office
Office européen des brevets

(11) EP 1 452 133 B1

# (12) EUROPEAN PATENT SPECIFICATION

(45) Date of publication and mention of the grant of the patent:
**06.01.2010 Bulletin 2010/01**

(51) Int Cl.:
***A61B 5/053*** *(2006.01)*

(21) Application number: **04004396.0**

(22) Date of filing: **26.02.2004**

(54) **Body composition estimation method and body composition measuring apparatus**

Verfahren und Vorrichtung zur Bestimmung der Körperzusammensetzung

Procédé et appareil pour estimer la composition du corps

(84) Designated Contracting States:
**DE FR GB**

(30) Priority: **28.02.2003 JP 2003052257**

(43) Date of publication of application:
**01.09.2004 Bulletin 2004/36**

(73) Proprietor: **TANITA CORPORATION
Tokyo (JP)**

(72) Inventor: **Takehara, Katsumi,
c/oTanita Corporation
Tokyo (JP)**

(74) Representative: **Grünecker, Kinkeldey,
Stockmair & Schwanhäusser
Anwaltssozietät
Leopoldstrasse 4
80802 München (DE)**

(56) References cited:
**EP-A- 0 865 763** **JP-A- 10 005 187**
**US-A- 5 615 689** **US-B1- 6 339 722**

- **PATENT ABSTRACTS OF JAPAN vol. 1997, no. 06, 30 June 1997 (1997-06-30) & JP 09 051884 A (SEKISUI CHEM CO LTD), 25 February 1997 (1997-02-25)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 452 133 B1

## Description

BACKGROUND OF THE INVENTION

(i) Field of the Invention

The present invention relates to an improvement in the accuracy of a bioelectrical impedance measuring method and to a body composition measuring apparatus based on the bioelectrical impedance measuring method.

(ii) Description of the Related Art

JP 10 005 187 A discloses a method and an apparatus according to the preamble of claims 1 and 13, respectively.

The bioelectrical impedance measuring method estimates a body composition based on the following principle.

It is assumed that a portion in a living body where an electric current passes through easily is represented by a cylindrical conductive material as shown in Fig. 1. Further, when the length, cross sectional area, resistivity and volume of the conductive material are represented by L, S, p and V, respectively, as shown in Fig. 1, a resistance R and a volume V between the upper and lower end faces of the conductive material are expressed as follows.

$$R = \rho L / S$$

$$V = SL$$

However, since the cross sectional area S is expressed as:

$$S = V/L,$$

the resistance R is expressed as follows.

$$R = \rho L^2 / V$$

Thus, the volume V is expressed as:

$$V = \rho L^2 / R.$$

Further, from these expressions, it is understood that a relationship represented by:

$$V \propto L^2 / R.$$

holds.

As described above, various body compositions are estimated by estimating the volume of the conductive material in the living body, that is, the volume V of water where an electric current passes through easily in the living body.

In the above model, it is assumed that the living body is a cylinder and water exists in the living body uniformly. However, in an actual living body, water existing therein comprises two types of compartments, i.e., one which has an extracellular fluid comprising an intercellular fluid and plasma and one which has an intracellular fluid surrounded by a cell membrane. Further, the cell membrane which surrounds the latter compartment having an intracellular fluid is considered as a very thin insulating material. A living body model having these two types of water compartments is called a compartment model. It is assumed that based on this model, a living body is represented by an equivalent circuit shown in Fig. 2 wherein Re is an extracellular fluid resistance, Ri is an intracellular fluid resistance and Cm is a cell membrane volume.

However, because an actual living body is a collection of various cells and it is difficult to represent the living body only by the lumped-constant equivalent circuit shown in Fig. 2, the Cole-Cole circular arc law is introduced so as to represent the electrical characteristics of the living body.

If it is assumed that the impedance vector locus of the living body conforms to the Cole-Cole circular arc law, a bioelectrical impedance vector Z at a given frequency can be expressed as follows:

$$Z(\omega) = R_\infty + (R_0 - R_\infty)/(1+(j\omega\tau)^\beta)$$

wherein $\omega$ denotes a measuring angular frequency (= $2\pi f$, f: measuring frequency), $\tau$ denotes a central relaxation constant of the Cole-Cole circular arc law, $\beta$ denotes a parameter representing distribution of relaxation time, R0 denotes a resistance value at a frequency of 0 Hz, and $R\infty$ denotes a resistance value at a frequency of $\infty$ Hz.

As for relationships between R0 and $R\infty$ and Re and Ri shown in Fig. 2, R0 which is an impedance value at a frequency of 0 Hz is merely a resistance value as shown by the equivalent circuit of Fig. 2.

$$\lim_{\omega \to 0} Z(\omega) = R_0 = R_e$$

Further, $R\infty$ which is an impedance value at a frequency of $\infty$ Hz is also merely a resistance value as in the above case.

$$\lim_{\omega \to 0} Z(\omega) = R_\infty = R_e R_i/(R_e+R_i)$$

When the frequency is 0 Hz, an electric current passes through the extracellular fluid compartment without passing through the intracellular fluid compartment. Therefore, a bioelectrical impedance vector value measured at a frequency of 0 Hz is a value based on the extracellular fluid.

Meanwhile, since a bioelectrical impedance vector value measured at a frequency of $\infty$ Hz passes through both the extracellular fluid compartment and the intracellular fluid compartment, it can be said to represent the amount of water in the whole body, i.e., a total body water amount.

Consequently, an extracellular fluid amount (ECW) or a total body water amount (TBW) is expressed as follows by use of R0 which is a resistance value when the frequency of a measuring electric current is 0 Hz and $R\infty$ which is a resistance value when the frequency is $\infty$ Hz.

$$ECW \propto L^2/R_0$$

$$TBW \propto L^2/R_\infty$$

Likewise, they are also expressed as follows by use of Re and Ri of Fig. 2.

$$ECW \propto L^2/R_e$$

$$TBW \propto L^2/(R_e R_i/(R_e+R_i))$$

Further, an intracellular fluid (ICW) is obtained by subtracting the extracellular fluid amount from the body water amount. Therefore, it can be expressed as follows.

$$ICW = TBW - ECW$$

As described above, the extracellular fluid amount, the total body water amount and the intracellular fluid amount can be estimated by use of the two compartment model and the Cole-Cole circular arc law.

Further, a lean body mass, a muscle amount, a body fat mass, and the proportions of the extracellular fluid amount, the total body water amount and the intracellular fluid amount in a body weight can also be estimated from the terms and reciprocals of the extracellular fluid amount, the total body water amount and the intracellular fluid amount and various parameters such as a body weight, a height, age and sex.

There is proposed an apparatus which calculates a body fat mass and a body water amount based on the values of R0 at a frequency of 0 Hz and R∞ at a frequency of ∞ Hz which have been obtained by measuring a bioelectrical impedance by use of the foregoing principle and measuring electric currents of multiple frequencies (for example, Patent Publication 1).

Further, there is disclosed a method that uses an impedance value including a reactance value in estimating the body cell amount, lean body weight and total body water amount of a person (for example, Patent Publication 2).

Patent Publication 1

Japanese Patent Laid-Open Publication No. 9-51884

Patent Publication 2

Specification of Japanese Patent No. 3330951

An electric current which passes through a living body at the time of measuring a bioelectrical impedance primarily passes through an extracellular fluid compartment and an intracellular fluid compartment which have an electrolyte component and have low resistivity. However, the intracellular fluid compartment is surrounded by a cell membrane considered as a very thin insulating film. This insulating film is represented as a condenser (Cm) in the equivalent circuit of Fig. 2. A direct current cannot pass through the insulating film, and its impedance changes in inverse proportion to frequency. Hence, the value of an electric current passing through the intracellular fluid compartment depends on the frequency of the passing electric current.

Meanwhile, the value of an electric current passing through the extracellular fluid compartment does not depend on the frequency of the passing electric current and shows a constant resistance value, as represented by extracellular fluid resistance (Re) in the equivalent circuit of Fig. 2.

A currently popular method of measuring body water or a body composition by use of a single frequency uses an electric current whose frequency is close to 50 kHz which is a frequency close to the characteristic frequency ($1/2\pi\tau$) by the Cole-Cole circular arc law. Within this frequency range, a measuring electric current passes through the extracellular fluid compartment sufficiently; however, only about 1/n (n = 2 to 9, for example) of electric current which passes at a frequency of ∞ passes through the intercellular fluid compartment due to the influence of the impedance of the cell membrane. However, it is possible to evaluate the body water and the body composition in thorough consideration of such electrical characteristics of a living body. However, because the influence of the intracellular fluid compartment on the bioelectrical impedance is smaller than that of the extracellular fluid compartment, various problems occur. Hereinafter, an example of the problems will be described.

Generally speaking, the extracellular fluid is constituted by plasma, a lymph fluid, an intercellular fluid and the like and moves relatively easily due to the influence of gravity or the like, while the intracellular fluid takes a relatively long time to move because it moves through a cell membrane. This implies that there is a possibility that only distribution of the extracellular fluid changes relatively easily in a short time depending on a body part to be measured or the position of a subject at the time of measuring the bioelectrical impedance. As described above, since the influence of the extracellular fluid compartment on the bioelectrical impedance is larger than that of the intracellular fluid compartment, such a change in the distribution of the extracellular fluid appears as a great change in the measurement value of the bioelectrical impedance and causes errors in estimation of body water or a body composition.

Further, it may also cause errors in the estimation of the body water or body composition even when the proportions of the extracellular fluid and the intracellular fluid of a subject are significantly different from those of a normal person. For example, it is assumed that athletes whose muscle amounts are significantly larger than those of ordinary persons have larger intracellular fluid compartments than those of ordinary persons according to the degree of development of the muscles. Thus, it is assumed that athletes have a larger proportion of intracellular fluid in body water than ordinary persons. However, due to the above reason, the intracellular fluid compartment is underestimated. Consequently, the intracellular fluid is underestimated, and a total body water amount is also underestimated.

Such a problem may occur not only in the measurement in the vicinity of the characteristic frequency but also in estimation of body water or a body composition by measurement of a bioelectrical impedance at a finite frequency wherein the extracellular fluid compartment and the intracellular fluid compartment are not evaluated equally.

The apparatus described in the above Japanese Patent Laid-Open Publication No. 9-51884 calculates resistance values at frequencies of 0 and ∞ by use of measuring signals of multiple frequencies and then calculates a specific bioelectrical impedance from these values. This calculation process takes time because the circular arc locus of the impedance must be determined. The value calculated above is a resistance value at a measuring frequency of 50 kHz. This value is calculated as the bioelectrical impedance value of a subject. This invention is intended to reduce the influence of aspiration in measuring the bioelectrical impedance and is not intended to suppresses changes in intracellular and extracellular fluids.

Meanwhile, the method described in the above Specification of Japanese Patent No. 3330951 uses a reactance value in estimating a human body composition by a bioelectrical impedance. The method uses a regression formula for calculating a body cell mass (BCM) and a reactance value Xcp. That is, the method directly substitutes the reactance value into the regression formula of the human body composition to be calculated. Further, this method deals with what can be measured by the bioelectrical impedance as a parallel electric circuit of an extracellular fluid and the body cell mass and does not evaluate an extracellular fluid compartment and an intracellular fluid compartment.

The present invention has been conceived in view of such problems. An object of the present invention is to make it possible to estimate body water, a body composition, etc., more accurately by correcting a measured bioelectrical impedance so as to suppress a change in the bioelectrical impedance which is caused by movements of intracellular and extracellular fluids and using the corrected value for estimating the body water, body composition, etc., at the time of estimation of the body water, body composition, etc., by measurement of the bioelectrical impedance.

SUMMARY OF THE INVENTION

A body composition estimation method of the present invention comprises correcting a parameter value of a measured bioelectrical impedance by use of a parameter representing an intracellular/extracellular fluid ratio which is included in the parameter value of the bioelectrical impedance measured at a given frequency and estimating a body composition and the like based on the corrected parameter value associated with the bioelectrical impedance. Thereby, it reduces the influence of a change in the distribution of an extracellular fluid which occurs in a relatively short time and estimates body water, the body composition and the like more accurately.

Further, in the body composition estimation method of the present invention, the given frequency is the frequency of the electric current applied to the living body for estimation of the body composition.

Further, in the body composition estimation method of the present invention, the given frequency is a frequency different from the frequency of the electric current applied to the living body for estimation of the body composition.

Further, in the body composition estimation method of the present invention, the parameter to be corrected of the bioelectrical impedance is any of the absolute value of the bioelectrical impedance, a bioelectrical impedance vector value or the resistance component value of the bioelectrical impedance vector which has heretofore been used for estimation of a body composition.

Further, in the body composition estimation method of the present invention, the parameter P' associated with the bioelectrical impedance which has been corrected by the parameter associated with the bioelectrical impedance which represents the intracellular/extracellular fluid ratio is calculated as follows:

$$P' = f(P,\alpha) = K \cdot P^A \cdot \alpha^B + C$$

wherein $f(P,\alpha)$ is a correction function represented by parameters P and $\alpha$, P' is the corrected parameter associated with the bioelectrical impedance, P is the measured parameter associated with the bioelectrical impedance, $\alpha$ is the parameter associated with the bioelectrical impedance which represents the intracellular/extracellular fluid ratio, and A, B, C and K are constants.

The body composition estimation method of the present invention makes more accurate estimations of body water, a body composition and the like based on the parameter associated with the bioelectrical impedance which has been calculated in accordance with the above expression.

Further, the parameter $\alpha$ associated with the bioelectrical impedance which is used in the body composition estimation method of the present invention and represents the intracellular/extracellular fluid ratio is expressed as follows by use of a phase difference $\phi$ between the waveform of the alternating current applied to the living body and the waveform of the measured voltage at the time of measurement of the bioelectrical impedance.

$$\alpha = 1/\phi$$

$$\alpha = 1/\tan(\phi)$$

Further, in the body composition estimation method of the present invention, the parameter $\alpha$ associated with the bioelectrical impedance which represents the intracellular/extracellular fluid ratio is expressed as follows by use of a parameter included in the parameter associated with the bioelectrical impedance to be corrected or a parameter associated with a bioelectrical impedance which is measured at other frequency.

$$\alpha = R/X$$

wherein R is the resistance component of the bioelectrical impedance, and X is the reactance component of the bioelectrical impedance.

Further, in the body composition estimation method of the present invention, the parameter $\alpha$ associated with the bioelectrical impedance which represents the intracellular/extracellular fluid ratio is expressed as follows by use of the absolute value of the bioelectrical impedance or the resistance component value of the bioelectrical impedance which is a parameter associated with a bioelectrical impedance at higher and lower frequencies than a measuring frequency for the parameter associated with the bioelectrical impedance to be corrected or either one of which is the parameter associated with the bioelectrical impedance to be corrected.

$$\alpha = P_{high}/P_{low}$$

$$\alpha = P_{low}/(P_{low}-P_{high})$$

$$\alpha = P_{high}/(P_{low}-P_{high})$$

wherein P_high is a parameter associated with a bioelectrical impedance at a higher frequency, and P_low is a parameter associated with a bioelectrical impedance at a lower frequency.

Further, in the body composition estimation method of the present invention, the parameter $\alpha$ associated with the bioelectrical impedance which represents the intracellular/extracellular fluid ratio is expressed as follows by a bioelectrical impedance value R0 at a frequency of 0 Hz and a bioelectrical impedance value Rinf at an infinite frequency which are determined from bioelectrical impedance values measured at a number of frequencies.

$$\alpha = Rinf/R0$$

Alternatively, the parameter $\alpha$ is expressed as follows by an extracellular fluid resistance value Re and an intracellular fluid resistance value Ri.

$$\alpha = Ri/Re$$

Further, a body composition measuring apparatus of the present invention comprises:

an electric current applying unit,
a voltage measuring unit,
a bioelectrical impedance computing unit,
a correcting unit, and

a body composition computing unit,

wherein

the electric current applying unit applies an electric current ) to a living body,
the voltage measuring unit measures a voltage,
the bioelectrical impedance computing unit computes a parameter associated with a bioelectrical impedance of a measured body part from the applied electric current and the measured voltage, the correcting unit corrects the parameter value associated with the measured bioelectrical impedance by use of a parameter representing an intracellular/extracellular fluid ratio which is included in the parameter value of the bioelectrical impedance measured at a given frequency, and
the body composition computing unit computes an index associated with a body composition based on the corrected parameter value associated with the bioelectrical impedance. Thereby, it reduces the influence of a change in the distribution of an extracellular fluid which occurs in a relatively short time and estimates body water, the body composition and the like more accurately.

Further, in the body composition measuring apparatus of the present invention, the given frequency is the frequency of the electric current applied to the living body for estimation of the body composition.
Further, in the body composition measuring apparatus of the present invention, the given frequency is a frequency different from the frequency of the electric current applied to the living body for estimation of the body composition.
Further, in the body composition measuring apparatus of the present invention, the parameter of the bioelectrical impedance which is corrected by the correcting unit is any of the absolute value of the bioelectrical impedance, a bioelectrical impedance vector value or the resistance component value of the bioelectrical impedance vector which has heretofore been used for estimation of a body composition.
Further, in the body composition measuring apparatus of the present invention, when the parameter associated with the bioelectrical impedance which has been corrected by the parameter associated with the bioelectrical impedance which represents the intracellular/extracellular fluid ratio is P', the correction of the parameter associated with the bioelectrical impedance in the correcting unit is made in accordance with the following correction expression:

$$P' = f(P,\alpha) = K \cdot P^{A} \cdot \alpha^{B} + C$$

wherein f(P,$\alpha$) is a correction function represented by parameters P and $\alpha$, P' is the corrected parameter associated with the bioelectrical impedance, P is the measured parameter associated with the bioelectrical impedance, $\alpha$ is the parameter associated with the bioelectrical impedance which represents the intracellular/extracellular fluid ratio, and A, B, C and K are constants. The body composition measuring apparatus of the present invention makes more accurate estimations of body water, a body composition and the like based on the calculated parameter associated with the bioelectrical impedance.
Further, in the body composition measuring apparatus of the present invention, the parameter $\alpha$ associated with the bioelectrical impedance which is used in the body composition measuring apparatus of the present invention and represents the intracellular/extracellular fluid ratio is expressed as follows by use of a phase difference $\phi$ between the waveform of the alternating current applied to the living body and the waveform of the measured voltage at the time of measurement of the bioelectrical impedance.

$$\alpha = 1/\phi$$

$$\alpha = 1/\tan(\phi)$$

Further, in the body composition measuring apparatus of the present invention, the parameter $\alpha$ associated with the bioelectrical impedance which represents the intracellular/extracellular fluid ratio is expressed as follows by use of a parameter included in the parameter associated with the bioelectrical impedance to be corrected or a parameter associated with a bioelectrical impedance which is measured at other frequency.

$$\alpha = R/X$$

wherein R is the resistance component of the bioelectrical impedance, and X is the reactance component of the bioelectrical impedance.

Further, in the body composition measuring apparatus of the present invention, the parameter $\alpha$ associated with the bioelectrical impedance which represents the intracellular/extracellular fluid ratio is expressed as follows by use of the absolute value of the bioelectrical impedance or the resistance component value of the bioelectrical impedance which is a parameter associated with a bioelectrical impedance at higher and lower frequencies than a measuring frequency for the parameter associated with the bioelectrical impedance to be corrected or either one of which is the parameter associated with the bioelectrical impedance to be corrected.

$$\alpha = P_{high}/P_{low}$$

$$\alpha = P_{low}/(P_{low}-P_{high})$$

$$\alpha = P_{high}/(P_{low}-P_{high})$$

wherein P_high is a parameter associated with a bioelectrical impedance at a higher frequency, and P_low is a parameter associated with a bioelectrical impedance at a lower frequency.

Further, in the body composition measuring apparatus of the present invention, the parameter $\alpha$ associated with the bioelectrical impedance which represents the intracellular/extracellular fluid ratio is expressed as follows by a bioelectrical impedance value R0 at a frequency of 0 Hz and a bioelectrical impedance value Rinf at an infinite frequency which are determined from bioelectrical impedance values measured at a number of frequencies.

$$\alpha = Rinf/R0$$

Alternatively, the parameter $\alpha$ is expressed as follows by an extracellular fluid resistance value Re and an intracellular fluid resistance value Ri.

$$\alpha = Ri/Re$$

BRIEF DESCRIPTION OF THE DRAWINGS

Fig. 1 is a diagram when a human body is assumed to be a cylinder.
Fig. 2 is a diagram showing an electrical equivalent circuit of an interstitial cell.
Fig. 3 is a diagram showing the vector locus of a bioelectrical impedance of a human body.
Fig. 4 is a graph showing a relationship in calculation of a lean body mass of an ordinary person between when a correction is made and when no correction is made in a bioelectrical impedance correction formula of the present invention.
Fig. 5 is a graph showing a relationship in calculation of a lean body mass of an athlete between when a correction is made and when no correction is made in the bioelectrical impedance correction formula of the present invention.
Fig. 6 is a graph showing changes with time in a bioelectrical impedance when a correction is made and when no correction is made in the bioelectrical impedance correction formula of the present invention.
Fig. 7 is an external perspective view of a body composition measuring apparatus which is an example of the present invention.
Fig. 8 is an internal block diagram of the body composition measuring apparatus which is an example of the present invention.
Fig. 9 is a flowchart of the body composition measuring apparatus which is an example of the present invention.

DETAILED DESCRIPTION OF THE PREFERRED EMBODIMENTS

To estimate a body composition by use of a bioelectrical impedance Z, a computation is made generally by substituting the body height of a subject, the absolute value |Z| of the bioelectrical impedance and parameters such as a body weight, sex and age into a regression formula. The following is an important term of the regression formula.

$$L^2/|Z| \quad \ldots \quad (1)$$

wherein L denotes the body height of a subject or the length of a body part to be measured, and I Z | denotes an absolute value of a measured bioelectrical impedance. Further, this term is called an impedance index.

Meanwhile, a measured bioelectrical impedance vector Z(ω) is expressed as follows according to the above Cole-Cole model.

$$Z(\omega) = R_\infty + (R_0 - R_\infty)/\{1+(j\omega\tau)^\beta\}$$
$$= R_\infty + (R_0 - R_\infty)/[1+(\omega\tau)^\beta \times \{\cos(\pi\beta/2)+j\sin(\pi\beta/2)\}]$$

wherein ω denotes a measuring angular frequency (= 2πf, f: measuring frequency), τ denotes a central relaxation constant of the circular arc law, β denotes a parameter representing distribution of relaxation time, R0 denotes a resistance value at a frequency of 0 Hz, and R∞ denotes a resistance value at a frequency of ∞ Hz.

As represented by the Cole-Cole model, when a bioelectrical impedance vector measured while a frequency is swept is plotted on a plane whose horizontal axis represents a resistance component R which is a real number component and vertical axis represents a volume component X which is an imaginary number component, its vector locus forms a circular arc as shown in Fig. 3. Although the imaginary axis component in this case is a negative value because the component is volume-based, the component will be dealt with as a positive number for the sake of convenience hereinafter.

In Fig. 3, O represents the coordinate origin, A and B represent intersections between the vector locus and the real axis, C represents the apex of the circular arc, D represents the center of the circle, and E represents an intersection between the line CD and the real axis. In this case, the point A represents a bioelectrical impedance value at a frequency of ∞, the point B represents a bioelectrical impedance value at a frequency of 0 Hz, and both of the points A and B have only a resistance value which is a real number component without an imaginary number component. A frequency at which the bioelectrical impedance value reaches the apex C of the circular arc is called a characteristic frequency, and an angular frequency at that time is expressed as follows.

$$\omega = 1/\tau$$

When the bioelectrical impedance value is broken down into the real axis component (resistance component) R and the imaginary axis component (reactance component) X, R and X are expressed as follows:

$$R = R_\infty + [(R_0 - R_\infty) \times \{1+(\omega\tau)^\beta \times \cos(\pi\beta/2)\}]/g(\omega,\tau,\beta)$$

$$X = [(R_0 - R_\infty) \times \{(\omega\tau)^\beta \times \sin(\pi\beta/2)\}]/g(\omega,\tau,\beta)$$

wherein

$$g(\omega,\tau,\beta) = 1+2(\omega\tau)^\beta \times \cos(\pi\beta/2)+\{(\omega\tau)^{2\beta}\}.$$

When a measuring angular frequency ω of 1/τ, i.e., the 5 characteristic frequency is considered, the above R and X are expressed as follows.

$$R = (R_0+R_\infty)/2$$

$$X = \{(R_0-R_\infty)/2\} \times [\sin(\pi\beta/2)/\{1+\cos(\pi\beta/2)\}]$$
$$= \{(R_0-R_\infty)/2\} \times \tan(\pi\beta/4)$$

The values of R and X in this case represent a distance between the coordinate origin and the point E and a distance between the points E and C shown in Fig. 3, respectively.

It is understood from the above description that body water, a body composition and the like can be estimated from both the absolute value |Z| of the bioelectrical impedance and the resistance component R which is its real axis component, although they comprise different parameters.

Therefore, in addition to the above impedance index:

$$L^2/|Z| \ ... \ (1),$$

body water, a body composition and the like can be estimated by:

$$L^2/R \ ... \ (1)'.$$

Hereinafter, this term will be called a resistance index.

These impedance index and resistance index has significant relationships with body water, a lean body mass, a muscle amount and the like in a living body, and they are important terms for estimating these data. Further, these indices are also important terms for estimating a fat mass, a fat percentage and the like.

However, in either case, in the measurement of a bioelectrical impedance at a frequency close to the characteristic frequency, influences of the above underestimation of the intracellular fluid compartment and the above change in the distribution of the extracellular fluid occur. Further, their influences may also occur in other bioelectrical impedance measurements at finite frequencies.

Further, this error produces a particularly significant influence when the body composition of a whole body is to be estimated from the resistance component of a specific part of the living body.

The present invention provides a method comprising correcting the absolute value |Z| of a measured bioelectrical impedance or a measured resistance component R by use of elements which are not reflected on the absolute value |Z| of the bioelectrical impedance or resistance component R obtained by the measurement of the bioelectrical impedance, i.e., data of an underestimated intracellular fluid compartment and estimating body water, a body composition and the like by use of an impedance index or resistance index using the absolute value |Z|' of the corrected bioelectrical impedance or the corrected resistance component R'; and an apparatus which estimates body water, a body composition and the like by use of the absolute value |Z|' of the corrected bioelectrical impedance or the corrected resistance component R'.

Hereinafter, the present invention will be described by use of a resistance index by a resistance component R which is a real axis component of a bioelectrical impedance vector.

In general, an impedance vector z(ω) is expressed as follows by use of a resistance component r and a reactance component x.

$$z(\omega) = r + jx(\omega)$$

Further, there exist the following relationships using the absolute value |z(ω)| of the impedance vector and a phase angle ϕ.

$$r = |z(\omega)|\cos\phi$$

$$x = |z(\omega)|\sin\phi$$

$$\tan(\phi) = x/r$$

Further, as described above, the resistance component R and the reactance component X are expressed as follows.

$$R = (R_0+R_\infty)/2$$

$$X = \{(R_0-R_\infty)/2\} \times \tan(\pi\beta/4)$$

Therefore, the following expression holds.

$$X/R = \tan(\pi\beta/4) \times (R_0-R_\infty)/(R_0+R_\infty)$$

Since the following expressions:

$$R0 = Re$$

$$R\infty = ReRi/(Re+Ri)$$

hold, X/R can also be expressed as follows.

$$X/R = \tan(\pi\beta/4) \times R_e/(R_e+2R_i)$$
$$= \tan(\pi\beta/4)/(1+2R_i/R_e)$$

Thus, the following expression holds.

$$R/X = (1+2R_i/R_e)\cot(\pi\beta/4)$$

Ri/Re in this expression represents relative data of the intracellular and extracellular fluid compartments. Hereinafter, this Ri/Re will be called an intracellular/extracellular fluid compartment ratio.

On this intracellular/extracellular fluid compartment ratio, the sizes of both compartments in a body part where a bioelectrical impedance is measured are reflected. In the case of an athlete having well-developed muscles, when the intracellular fluid compartment is large, the intracellular/extracellular fluid compartment ratio is small. On the other hand, the smaller the intracellular fluid compartment becomes, the larger the intracellular/extracellular fluid compartment ratio becomes. Meanwhile, when the extracellular fluid compartment is large, the intracellular/extracellular fluid compartment ratio is large, while when the extracellular fluid compartment is small, the intracellular/extracellular fluid compartment ratio is small.

However, it is not conceivable that the intracellular fluid compartment of a living body changes within a short time under general circumstances. It is conceived that the intracellular fluid compartment changes gradually over a long time by training, aging and the like. Therefore, it may be assumed that the intracellular fluid compartment remains unchanged within a limited time period. As for the extracellular fluid compartment, however, since a change may occur in uniform distribution of the extracellular fluid in a very short time as described above, the extracellular fluid compartment may change drastically.

In the present invention, the absolute value of a bioelectrical impedance or the resistance component of the bioelectrical impedance which is a parameter associated with the measured bioelectrical impedance is corrected by use of a parameter associated with the bioelectrical impedance which includes the above intracellular/extracellular fluid compartment ratio in accordance with the following expression. Hereinafter, the intracellular/extracellular fluid compartment ratio and the ratio between the intracellular and extracellular fluids will be used synonymously with each other and will not be differentiated.

$$P' = f(P,\alpha) = K \cdot P^A \cdot \alpha^B + C$$

wherein f(P,α) is a correction function represented by parameters P and α, P' is a parameter associated with a corrected bioelectrical impedance, P is a parameter associated with a measured bioelectrical impedance, α is a parameter associated with a bioelectrical impedance which represents an intracellular/extracellular fluid ratio, and A, B, C and K are constants.

Further, by use of the parameter value associated with the corrected bioelectrical impedance, the above impedance index or resistance index is calculated, and based on the calculated index, body water and a body composition are estimated.

The parameter having the intracellular/extracellular fluid compartment ratio for correcting the parameter associated with the bioelectrical impedance, when the phase angle of the bioelectrical impedance is ϕ, is expressed as follows.

$$R/X = \cot(\phi)$$

Considering that the phase angle of the bioelectrical impedance is generally smaller than 10°, it is conceivable that the following expression holds.

$$\cot(\phi) \fallingdotseq 1/\phi$$

Thus,

$$\cot(\phi)$$

and

$$1/\phi.$$

An extracellular fluid resistance value Re and an intracellular fluid resistance value Ri calculated from the results of measurement of a bioelectrical impedance at multiple frequencies have the following relationship.

$$Ri/Re$$

A bioelectrical impedance R0 at a frequency of 0 Hz and a bioelectrical impedance R∞ at a frequency of ∞ calculated from the results of measurement of a bioelectrical impedance at multiple frequencies have the following relationship.

Rinf/R0

∵

$$R_{inf}/R_0 = \{R_eR_i/(R_e+R_i)\}/R_e$$
$$= 1/\{1+(R_i/R_e)\}$$

R0/(R0-Rinf)

∵

$$R_0/(R_0-R_{inf}) = 1 + (R_i/R_e)$$

Rinf/(R0-Rinf)

∵

$$R_{inf}/(R_0-R_{inf}) = R_i/R_e$$

When both or either of the above R0 and Rinf are approximated by use of the absolute value |Z| of a bioelectrical impedance vector or the resistance component R of the bioelectrical impedance vector which is a parameter associated with a bioelectrical impedance at higher and lower frequencies than a measuring frequency for the parameter associated with the bioelectrical impedance to be corrected or either one of which is the parameter associated with the bioelectrical impedance to be corrected, the following expressions hold.

$$P_{_high}/P_{_low}$$

$$P_{_low}/(P_{_low}-P_{_high})$$

$$P_{_high}/(P_{_low}-P_{_high})$$

wherein $P_{_high}$ is a parameter associated with a bioelectrical impedance at a higher frequency, and $P_{_low}$ is a parameter associated with a bioelectrical impedance at a lower frequency.

Examples

Hereinafter, the effectiveness of the bioelectrical impedance measuring method according to the present invention and an example of the bioelectrical impedance measuring apparatus according to the present invention will be described with reference to the drawings.

To examine the effectiveness of the bioelectrical impedance measuring method according to the present invention, the present inventor conducted an experiment for examining a difference in the result between before and after correction made by the bioelectrical impedance measuring method of the present invention by use of a bioelectrical impedance. As a measuring electric current, an alternating current signal of 50 kHz which was close to the characteristic frequency was employed. The electric current for measuring a bioelectrical impedance passed between both feet, and an electric potential difference was also detected between the feet.

This examination experiment shows the result of simply substituting the absolute value of a measured impedance value into the expression (1) which is an impedance index which is the most important element in estimation of a body composition and the result of correcting the absolute value of the measured bioelectrical impedance by use of the following expression (2) and substituting the corrected value into the expression (1) which is the impedance index. In this case, the former is defined as a pre-correction value, and the latter is defined as a post-correction value.

By use of R/X as a parameter representing an intracellular/extracellular fluid ratio, the absolute value P of a measured bioelectrical impedance was corrected by the following expression.

$$P' = f(P, R, X) = K_1 (P)^{A_1} (R/X)^{B_1} + C_1 \quad \ldots \quad (2)$$

wherein R is a real axis component (resistance component) of a bioelectrical impedance, X is an imaginary axis component (reactance component) of the bioelectrical impedance, and $A_1$, $B_1$, $C_1$ and $K_1$ are constants.

Fig. 4 and Fig. 5 show lean body masses obtained by dual energy X-ray absorptiometry (DXA) on the horizontal axis and pre-correction values and post-correction values obtained by the impedance index and normalized by their corresponding maximum values on the vertical axis. "O" represents the pre-correction values, and "x" represents the post-correction values. Further, Fig. 4 shows pre-correction values and post-correction values for ordinary persons, while Fig. 5 shows pre-correction values and post-correction values for athletes.

Referring to the data for ordinary persons in Fig. 4, differences are hardly seen in comparisons of the post-correction values with the pre-correction values. This is because the parameters for the correction expression are prepared in accordance with the data for ordinary persons. No differences between the pre-correction and post-correction data indicates that the present invention can be applied to an unspecified number of subjects having a general intracellular/extracellular compartment ratio.

As for the data for athletes in Fig. 5, when they are compared with the results for ordinary persons in Fig. 4, there are distinct differences, and it is understood that the values calculated by the absolute values of bioelectrical impedances before correction are low on the whole and lean body masses are underestimated. The larger the lean body masses measured by DEXA, the more noticeable this tendency becomes. This is ascribable to the aforementioned fact that the extracellular fluid compartment and the intracellular fluid compartment contribute to measured bioelectrical impedance values to different degrees. It is understood that by making the correction according to the present invention, the tendency of underestimation of the lean body masses for athletes has been improved and values close to proper lean body masses have been calculated. Therefore, the correction according to the present invention can make estimated values closer to proper values even when the intracellular/extracellular compartment ratio is significantly different from that of an ordinary person.

The above is the results of examining the application of the present invention when subjects are in normal conditions or in conditions close to the normal conditions.

Next, an examination is made on when the extracellular fluid compartment of a subject changes. This corresponds to the aforementioned case where the distribution of an extracellular fluid changes in a relatively short time. Fig. 6 is a graph showing, in chronological order, data calculated from values before and after the correction according to the present invention is made, by measuring a change in the bioelectrical impedance of a subject a few times per day over four days.

The horizontal axis represents time, and the vertical axis represents rates of change when the first values before and after the correction are 100. Further, "O" represents pre-correction values, and "x" represents post-correction values.

Changes in the graph of the pre-correction values imply that the measured bioelectrical impedance value changes during a day. The reason is as follows. That is, the distribution of water is uniform immediately after the subject wakes up because the subject has kept lying down by then, and the legs have a low water content, so that a measured bioelectrical impedance becomes high. However, when the subject starts a normal life after waking up, migration of the extracellular fluid occurs due to the influence of gravity, whereby the extracellular fluid in the legs which are body parts where the bioelectrical impedance is measured in this examination experiment increases, so that a low bioelectrical impedance value is obtained. The result of substituting the pre-correction absolute value of the bioelectrical impedance into the impedance index as in the foregoing examination experiment corresponds to the changes in the graph of the pre-correction values. Meanwhile, when the graph of the post-correction values is examined, changes assumed to be caused by migration of the extracellular fluid are hardly seen. Thus, by performing the correction described in the present invention on the absolute value of the measured bioelectrical impedance and estimating a body composition based on the corrected value, the influence of migration of the extracellular fluid can be reduced, and changes in body water and a body composition calculated in a day which are called "circadian rhythms" can be kept very low.

Next, an example of a body composition measuring apparatus using the bioelectrical impedance measuring method according to the present invention will be described.

Fig. 7 is an external view of the body composition measuring apparatus, and Fig. 8 is a block diagram for illustrating electrical connections of the apparatus.

Fig. 7 is an external perspective view of the body composition measuring apparatus which is an example of the present invention. The measuring apparatus 1 has nearly L-shaped. Its lower portion is constituted by a scale 2. The scale 2 is a known device and has electrode portions 3 and 4 on a platform 2a on which a subject stands on to measure his weight. The electrode portions 3 and 4 make contact with the bottoms of both feet of the subject. The electrode portions 3 and 4 comprise current supplying electrodes 3a and 4a for supplying an electric current and voltage measuring electrodes 3b and 4b for measuring a voltage.

Further, the measuring apparatus 1 has an operation box 5 on its top surface. This operation box 5 comprises an input unit 6 which is input means for inputting various physical data and comprises a number of keys including a power switch and numeric keys, a display unit 7 which is display means comprising an LCD for displaying the results of measurements, and a printing unit 8 which prints the results of measurements on paper and ejects the paper.

Further, to the operation box 5, electrode grips 13 and 14 for hands are connected via codes 15 and 16. The electrode grips 13 and 14 comprise current supplying electrodes 13a and 14a for supplying an electric current and voltage measuring electrodes 13b and 14b for measuring a voltage. The electrode grips 13 and 14 are hooked on hooks 17 which are provided on both sides of the operation box 5 except for when they are used for measurement.

Fig. 8 is an internal electrical block diagram of the measuring apparatus 1. Eight electrodes which are current applying means and voltage measuring means, i.e., electrodes 3a, 3b, 4a, 4b, 13a, 13b, 14a and 14b which make contact with both hands and feet, are connected to an electrode switching unit 20. The electrode switching unit 20 is connected to an arithmetic and control unit 23 which is control means via a current supplying unit 21 and a voltage measuring unit 22. The arithmetic and control unit 23 has a microcomputer (CPU) and is not only bioelectrical impedance computation means for computing a bioelectrical impedance from an applied electric current and a measured voltage but also correction means for correcting the computed bioelectrical impedance. Further, it is also body composition computation means for computing an index related to the composition of a living body and performs various other computations and controls. A storage unit 24 which is storage means for storing various data and comprises a memory or a register and a body weight measuring unit 26 which measures the body weight of a subject are connected to the arithmetic and control unit 23. Further, the input unit 6, the display unit 7 and the printing unit 8 are connected to the arithmetic and control unit 23. A power unit 28 supplies electric power to the arithmetic and control unit 23 and other units.

Next, the operation of the body composition measuring apparatus will be described.

Fig. 9 is a flowchart showing the operation of a body composition measuring apparatus 1.

At the press of the power switch of the input unit 6 (STEP S1), the apparatus is initialized (STEP S2). Thereby, the apparatus enters a waiting mode to accept the next input by a switch (STEP S3). Then, at the press of a numeric key of the input unit 6 (STEP S4), it is checked whether data about personal parameters are stored in a memory area in the storage unit 24 based on the corresponding number (STEP S5).

When the personal parameters are stored, the personal parameters are read from the storage unit 24 and displayed on the display unit 7, and it is then checked whether a switching key has been pressed (STEP S6).

When the personal parameters are not stored in STEP S4 or the switching key has been pressed in STEP S5, the apparatus enters a mode to wait for inputs of the personal parameters. A user enters personal parameters such as a body height, age and sex by use of the numeric keys of the input unit 6 (STEP S7).

When the personal parameters are entered, a body weight is measured (STEP S8). When the user stands on the scale 2, the body weight measuring unit 26 detects a load and measures the weight of the user.

Then, a bioelectrical impedance is measured (STEP S9).

A bioelectrical impedance between both hands is measured. The electrode switching unit 20 is switched by a signal from the arithmetic and control unit 23, whereby an alternating electric current is supplied from the current supplying unit 21 to between the electrodes 13a and 14a, and a voltage is measured on the electrodes 13b and 14b by the voltage measuring unit 22. At that time, a phase difference is determined from a voltage waveform produced when the applied current is passed through a reference resistance and the alternating waveform of the measured voltage in the measured body part of the living body, and the bioelectrical impedance value is corrected by the phase difference and the absolute value P of the measured bioelectrical impedance. As shown in Fig. 2, the phase difference occurs because a cell membrane in a living body has a volume component, and its size varies according to an intracellular/extracellular fluid ratio as can be understood from a fact that the equivalent circuit of a living body model can be represented as a parallel circuit of an extracellular fluid resistance and an intracellular fluid resistance. As for calculation of a parameter associated with the bioelectrical impedance to be corrected, the corrected value P' of the bioelectrical impedance is calculated by use of $1/\phi$ as a parameter for the intracellular/extracellular fluid ratio in accordance with the following expression:

$$P' = f(P,\phi) = K_2(P)^{A_2}(1/\phi)^{B_2} + C_2 \ldots (3)$$

wherein $\phi$ is a phase difference, and $A_2$, $B_2$, $C_2$ and $K_2$ are constants (STEP S10).

Then, a bioelectrical impedance between both feet is measured. An electric current is passed between the electrodes 3a and 4a, and a voltage is measured between the electrodes 3b and 4b.

Then, a bioelectrical impedance through the trunk is measured. An electric current is passed between the electrodes 14a and 4a, and a voltage is measured between the electrodes 13b and 3b.

After completion of measurement of the bioelectrical impedance in each body part, the body composition of the subject is calculated. The body composition is calculated by use of the corrected bioelectrical impedance values P'.

The body composition is calculated by use of the corrected bioelectrical impedance values, the set and stored personal parameters and the measured body weight value (STEP S11). The body composition such as a percent of body fat, a body water amount and a muscle amount to be calculated can be estimated from bioelectrical impedance values and physical parameters such as a body height and a body weight, and descriptions of calculations thereof are omitted since their calculations are conventionally known techniques.

The result of calculating the body composition is displayed on the display unit 7 (STEP S12). Thereafter, the apparatus returns to the key-input waiting mode of STEP S3.

When a personal key has not been pressed in STEP S4, it is determined whether the power switch has been pressed (STEP S13). When it is determined that the power switch has been pressed, the power is turn off, and the operation of the apparatus is ended completely (STEP S14).

Although the upper body including both hands, the lower body including both feet and the trunk have been described as body parts where a bioelectrical impedance is measured in this apparatus, the bioelectrical impedance measuring method of the present invention is not limited to measurements at specific body parts and can be applied to the measurement of the bioelectrical impedance at any body parts of a living body.

In the above example, the parameter associated with the phase difference is used. An example associated with other parameter will be described hereinafter.

Two measuring frequencies, i.e., f_high and f_low which is sufficiently lower than f_high, are conceived. When f_high is a sufficiently high frequency which passes through intracellular/extracellular fluids, its resistance component Rf_high can be a parameter for estimating total body water. Further, f_low which is sufficiently lower than f_high estimates the extracellular fluid more largely than f_high. Therefore, when viewed from Rf_high, Rf_low can be considered as a parameter for estimating the extracellular fluid.

Consequently, the resistance components Rf_high/Rf_low of the corresponding frequencies can be a biological parameter representing the ratio between the extracellular fluid and total body water.

How this parameter changes is conceived for an athlete and an ordinary person.

Athlete: More Muscle → Lower Extracellular Fluid Amount/Body Water Amount → Lower Rf_high/Rf_low
Ordinary Person: Less Muscle → Higher Extracellular Fluid Amount/Body Water Amount → Higher Rf_high/Rf_low

Thus, multiplying the resistance component R by this parameter makes the resistance component R smaller for those with a smaller proportion of the extracellular fluid amount in the total body water amount. That is, it is determined that those with more muscles have more body water.

Further, when a change in the extracellular fluid of a person is conceived on the assumption that an intracellular fluid does not change, the following are conceivable.

Extracellular Fluid Decreased (Resistance Component R Increased) → Lower Extracellular Fluid Amount/Body Water Amount → Rf_high/Rf_low Decreased

Extracellular Fluid Increased (Resistance Component R Decreased) → Higher Extracellular Fluid Amount/Body Water Amount → Rf_high/Rf_low Increased

Thus, this parameter Rf_high/Rf_low is a parameter which acts in an reverse direction to an increase/decrease in the resistance component R by an increase/decrease in the extracellular fluid. Although the above description has been given by use of the resistance component of the bioelectrical impedance vector, a parameter P' associated with a bioelectrical impedance to be corrected can be defined as follows when the above resistance component is replaced by a parameter P associated with the bioelectrical impedance which includes the absolute value |Z| of the bioelectrical impedance.

$$P' = K_3(P)^{A_3}(P_{f_high}/P_{f_low})^{B_3} + C_3$$

wherein $P_{_high}$ is a parameter associated with a bioelectrical impedance at a higher frequency, $P_{_low}$ is a parameter associated with a bioelectrical impedance at a lower frequency, and $A_3$, $B_3$, $C_3$ and $K_3$ are constants.

Next, another biological parameter reflecting the intracellular/extracellular fluid ratio of a person will be described.

In accordance with the Cole-Cole model based on a multifrequency method, resistance values related to the water amount of a living body such as Re representing an extracellular fluid, Rinf representing body water and Ri representing an extracellular fluid by Re and Rinf can be determined. Descriptions of calculations of the resistance values R0 and Rinf by multifrequency measuring signals will be omitted since they are described in the above Japanese Patent Laid-Open Publication No. 9-51884.

Since these resistance values represent an extracellular fluid amount, a total body water amount and an intracellular fluid amount, the same thing as said with respect to the above example can be said.

Accordingly, Rinf/Re and Ri/Re are parameters which work in the same manner as those in the above example. Therefore, even if corrections are made in accordance with the following expressions:

$$P' = K_4(P)^{A_4}(R_{inf}/R_e)^{B_4} + C_4$$

$$P' = K_5(P)^{A_5}(R_i/R_e)^{B_5} + C_5$$

wherein An, Bn, Cn and Kn are constants,
It is conceived that an improvement in the accuracy of measurement of a bioelectrical impedance can also be expected as in the above case.

As described in the above examples of the present invention, the present invention corrects a portion in a measured bioelectrical impedance which changes according to a change in an intracellular/extracellular fluid ratio by use of a parameter associated therewith. The parameter is not limited to those described above. For example, a number of the above correction parameters may be used in combination, and the following correction:

$$P' = f(P,\alpha,\beta\ \ldots) = K1\cdot P^A\cdot\alpha^B\cdot\beta^C\ \ldots + K3$$

or

$$P' = f(P,\alpha,\beta\ \ldots) = K1\cdot P^A\cdot(K11\alpha^B + K12\beta^C + \ldots + K2) + K3$$

wherein f(P,α,β...) is a correction function represented by parameters P, α and β, P' is a parameter associated with a corrected bioelectrical impedance, P is a parameter associated with a corrected bioelectrical impedance, α, β, ... are 5 parameters associated with a bioelectrical impedance which represent an intracellular/extracellular fluid ratio, A, B and C are parameters (constants) for conforming to a living body, K1, K2 and K3 are constants, and K11, K12, ... are also constants, is also possible.

Further, in the above descriptions of the body composition estimation method and body composition measuring apparatus of the present invention, the absolute value |Z| of a bioelectrical impedance vector or the resistance component R of the bioelectrical impedance vector is used as a parameter associated with a bioelectrical impedance to be corrected. However, even if the parameter associated with the bioelectrical impedance is neither the absolute value |Z| nor the resistance component R, the present invention which makes a correction by use of a parameter having an intracellular/extracellular fluid compartment ratio is still applicable.

Further, in the above descriptions of the body composition estimation method and body composition measuring apparatus of the present invention, parameters associated with bioelectrical impedances between both hands, between both feet and through the trunk are corrected by use of the electrodes for both hands and the electrodes for both feet. The present invention is not limited to this particular constitution. The present invention may also be constituted such that a bioelectrical impedance in a specific body part such as a hand, a foot, the right half body or the left half body is measured and a parameter associated with the bioelectrical impedance is corrected, and the body part where the bioelectrical impedance is measured is not limited.

The body composition estimation method and body composition measuring apparatus of the present invention correct a parameter associated with a bioelectrical impedance by use of a parameter representing an intracellular/extracellular fluid ratio. Thereby, a change in an impedance caused by a change in the extracellular fluid when a certain body water distribution state is taken as a standard can be suppressed. This implies that a change in a bioelectrical impedance called "circadian rhythms" can be controlled. The value of a parameter associated with a bioelectrical impedance to be calculated becomes a value free from the influence of a change in the intracellular/extracellular fluids, and a body composition is calculated more accurately based on the parameter.

Further, the body composition estimation method and body composition measuring apparatus of the present invention can calculate a phase difference from the waveform of an electric current to be applied and the waveform of a measured voltage easily when the phase difference is used as a parameter representing an intracellular/extracellular fluid ratio and can correct a bioelectrical impedance easily.

## Claims

1. A body composition estimation method comprising calculating a parameter of a bioelectrical impedance in a body part to be measured, from a parameter value of an electric current to be applied to a living body and a parameter value of a measured voltage, wherein
a body composition is estimated,
**characterized in that**
a parameter value of a bioelectrical impedance measured at a given frequency is corrected by use of a parameter representing an intracellular / extracellular fluid ratio, and
the body composition is estimated based on the corrected parameter value,
wherein when the parameter associated with the bioelectrical impedance which is corrected by the parameter associated with the bioelectrical impedance which represents the intracellular / extracellular fluid ratio is P', P' is calculated in accordance with the following correction expression:

$$P' = f(P,\alpha) = K \cdot P^{A} \cdot \alpha^{B} + C$$

wherein f(P,α) is a correction function represented by parameters P and α, P' is the corrected parameter associated with the bioelectrical impedance, P is the measured parameter associated with the bioelectrical impedance, α is the parameter associated with the bioelectrical impedance which represents the intracellular / extracellular fluid ratio, and A, B, C and K are constants.

2. The method of claim 1, wherein the given frequency is the frequency of the electric current applied to the living body for estimation of the body composition.

3. The method of claim 1, wherein the given frequency is a frequency different from the frequency of the electric current applied to the living body for estimation of the body composition.

4. The method of claim 1, wherein the parameter to be corrected of the bioelectrical impedance is any of the absolute value of the bioelectrical impedance, a bioelectrical impedance vector value or the resistance component value of the bioelectrical impedance vector.

5. The method of any one of claims 1 to 4, wherein the parameter α associated with the bioelectrical impedance which represents the intracellular / extracellular fluid ratio is expressed as follows by use of a phase difference Φ between the waveform of the alternating current applied to the living body and the waveform of the measured voltage at the time of measurement of the bioelectrical impedance:

$$\alpha = 1/\Phi .$$

6. The method of any one of claims 1 to 4, wherein the parameter α associated with the bioelectrical impedance which represents the intracellular / extracellular fluid ratio is expressed as follows by use of a phase difference Φ between the waveform of the alternating current applied to the living body and the waveform of the measured voltage at the time of measurement of the bioelectrical impedance:

$$\alpha = 1/\tan(\Phi) .$$

**7.** The method of any one of claims 1 to 4, wherein the parameter alpha associated with the bioelectrical impedance which represents the intracellular / extracellular fluid ratio is expressed as follows by use of a parameter included in the parameter associated with the bioelectrical impedance to be corrected or a parameter associated with a bioelectrical impedance which is measured at other frequency:

$$\alpha = R/X$$

wherein R is the resistance component of the bioelectrical impedance, and X is the reactance component of the bioelectrical impedance.

**8.** The method of any one of claims 1 to 4, wherein the parameter $\alpha$ associated with the bioelectrical impedance which represents the intracellular / extracellular fluid ratio is expressed as follows by use of the absolute value of the bioelectrical impedance or the resistance component value of the bioelectrical impedance which is a parameter associated with a bioelectrical impedance at higher and lower frequencies than a measuring frequency for the parameter associated with the bioelectrical impedance to be corrected or either one of which is the parameter associated with the bioelectrical impedance to be corrected:

$$\alpha = P_{_high}/P_{_low}$$

wherein $P_{_high}$ is a parameter associated with a bioelectrical impedance at a higher frequency, and $P_{_low}$ is a parameter associated with a bioelectrical impedance at a lower frequency.

**9.** The method of any one of claims 1 to 4, wherein the parameter $\alpha$ associated with the bioelectrical impedance which represents the intracellular / extracellular fluid ratio is expressed as follows by use of the absolute value of the bioelectrical impedance or the resistance component value of the bioelectrical impedance which is a parameter associated with a bioelectrical impedance at higher and lower frequencies than a measuring frequency for the parameter associated with the bioelectrical impedance to be corrected or either one of which is the parameter associated with the bioelectrical impedance to be corrected:

$$\alpha = P_{_low}/ (P_{_low}-P_{_hiqh})$$

wherein $P_{_high}$ is a parameter associated with a bioelectrical impedance at a higher frequency, and $P_{_low}$ is a parameter associated with a bioelectrical impedance at a lower frequency.

**10.** The method of any one of claims 1 to 4, wherein the parameter $\alpha$ associated with the bioelectrical impedance which represents the intracellular / extracellular fluid ratio is expressed as follows by use of the absolute value of the bioelectrical impedance or the resistance component value of the bioelectrical impedance which is a parameter associated with a bioelectrical impedance at higher and lower frequencies than a measuring frequency for the parameter associated with the bioelectrical impedance to be corrected or either one of which is the parameter associated with the bioelectrical impedance to be corrected:

$$\alpha = P_{_high}/(P_{_low}-P_{_high})$$

wherein $P_{_high}$ is a parameter associated with a bioelectrical impedance at a higher frequency, and $P_{_low}$ is a parameter associated with a bioelectrical impedance at a lower frequency.

**11.** The method of any one of claims 1 to 4, wherein the parameter $\alpha$ associated with the bioelectrical impedance which represents the intracellular / extracellular fluid ratio is expressed as follows by a bioelectrical impedance value R0 at a frequency of 0 Hz and a bioelectrical impedance value Rinf at an infinite frequency which are determined from bioelectrical impedance values measured at a number of frequencies:

$$\alpha = Rinf/R0 .$$

**12.** The method of any one of claims 1 to 4, wherein the parameter $\alpha$ associated with the bioelectrical impedance which represents the intracellular/extracellular fluid ratio is expressed as follows by use of an intracellular fluid resistance value Ri and an extracellular fluid resistance value Re which are calculated based on a bioelectrical impedance value R0 at a frequency of 0 Hz and a bioelectrical impedance value Rinf at an infinite frequency which are determined from bioelectrical impedance values measured at a number of frequencies:

$$\alpha = Ri/Re .$$

**13.** A body composition measuring apparatus (1) comprising:

an electric current applying unit (21),
a voltage measuring unit (22),
a bioelectrical impedance computing unit (23),
a correcting unit (23), and
a body composition computing unit (23),
wherein
the electric current applying unit (21) applies an electric current to a living body,
the voltage measuring unit (22) measures a voltage,
the bioelectrical impedance computing unit (23) computes a parameter associated with a bioelectrical impedance of a measured body part from the applied electric current and the measured voltage,
**characterized in that**
the correcting unit (23) corrects the parameter value associated with the measured bioelectrical impedance at a given frequency by use of a parameter representing an intracellular / extracellular fluid ratio, and the body composition computing unit (23) computes an index associated with a body composition based on the corrected parameter value associated with the bioelectrical impedance,
wherein when the parameter associated with the bioelectrical impedance which has been corrected by the parameter associated with the bioelectrical impedance which represents the intracellular / extracellular fluid ratio is P', the correction of the parameter associated with the bioelectrical impedance in the correcting unit (23) is made in accordance with the following correction expression:

$$P' = f(P,\alpha) = K \cdot P^{A} \cdot \alpha^{B} + C$$

wherein $f(P,\alpha)$ is a correction function represented by parameters P and $\alpha$, P' is the corrected parameter associated with the bioelectrical impedance, P is the measured parameter associated with the bioelectrical impedance, $\alpha$ is the parameter associated with the bioelectrical impedance which represents the intracellular / extracellular fluid ratio, and A, B, C and K are constants.

**14.** The apparatus of claim 13, wherein the given frequency is the frequency of the electric current applied to the living body for estimation of the body composition.

**15.** The apparatus of claim 13, wherein the given frequency is a frequency different from the frequency of the electric current applied to the living body for estimation of the body composition.

**16.** The apparatus of claim 13, wherein the parameter of the bioelectrical impedance which is corrected by the correcting unit is any of the absolute value of the bioelectrical impedance, a bioelectrical impedance vector value or the resistance component value of the bioelectrical impedance vector.

**17.** The apparatus of any one of claims 13 to 16, wherein the parameter $\alpha$ associated with the bioelectrical impedance which represents the intracellular / extracellular fluid ratio is expressed as follows by use of a phase difference $\Phi$ between the waveform of the alternating current applied to the living body and the waveform of the measured voltage at the time of measurement of the bioelectrical impedance:

$$\alpha = 1/\Phi .$$

**18.** The apparatus of any one of claims 13 to 16, wherein the parameter $\alpha$ associated with the bioelectrical impedance which represents the intracellular / extracellular fluid ratio is expressed as follows by use of a phase difference $\Phi$ between the waveform of the alternating current applied to the living body and the waveform of the measured voltage at the time of measurement of the bioelectrical impedance:

$$\alpha = 1/\tan(\Phi) .$$

**19.** The apparatus of any one of claims 13 to 16, wherein the parameter alpha associated with the bioelectrical impedance which represents the intracellular / extracellular fluid ratio is expressed as follows by use of a parameter included in the parameter associated with the bioelectrical impedance to be corrected or a parameter associated with a bioelectrical impedance which is measured at other frequency:

$$\alpha = R/X$$

wherein R is the resistance component of the bioelectrical impedance, and X is the reactance component of the bioelectrical impedance.

**20.** The apparatus of any one of claims 13 to 16, wherein the parameter $\alpha$ associated with the bioelectrical impedance which represents the intracellular / extracellular fluid ratio is expressed as follows by use of the absolute value of the bioelectrical impedance or the resistance component value of the bioelectrical impedance which is a parameter associated with a bioelectrical impedance at higher and lower frequencies than a measuring frequency for the parameter associated with the bioelectrical impedance to be corrected or either one of which is the parameter associated with the bioelectrical impedance to be corrected:

$$\alpha = P_{_high}/P_{_low}$$

wherein $P_{_high}$ is a parameter associated with a bioelectrical impedance at a higher frequency, and $P_{_low}$ is a parameter associated with a bioelectrical impedance at a lower frequency.

**21.** The apparatus of any one of claims 13 to 16, wherein the parameter $\alpha$ associated with the bioelectrical impedance which represents the intracellular / extracellular fluid ratio is expressed as follows by use of the absolute value of the bioelectrical impedance or the resistance component value of the bioelectrical impedance which is a parameter associated with a bioelectrical impedance at higher and lower frequencies than a measuring frequency for the parameter associated with the bioelectrical impedance to be corrected or either one of which is the parameter associated with the bioelectrical impedance to be corrected:

$$\alpha = P_{_low} / (P_{_low} - P_{_hiqh})$$

wherein $P_{_high}$ is a parameter associated with a bioelectrical impedance at a higher frequency, and $P_{_low}$ is a parameter associated with a bioelectrical impedance at a lower frequency.

**22.** The apparatus of any one of claims 13 to 16, wherein the parameter $\alpha$ associated with the bioelectrical impedance which represents the intracellular / extracellular fluid ratio is expressed as follows by use of the absolute value of the bioelectrical impedance or the resistance component value of the bioelectrical impedance which is a parameter associated with a bioelectrical impedance at higher and lower frequencies than a measuring frequency for the parameter associated with the bioelectrical impedance to be corrected or either one of which is the parameter associated with the bioelectrical impedance to be corrected:

$$\alpha = P_{_high} / (P_{_low} - P_{_high})$$

wherein $P_{_high}$ is a parameter associated with a bioelectrical impedance at a higher frequency, and $P_{_low}$ is a parameter associated with a bioelectrical impedance at a lower frequency.

**23.** The apparatus of any one of claims 13 to 16, wherein the parameter $\alpha$ associated with the bioelectrical impedance which represents the intracellular / extracellular fluid ratio is expressed as follows by a bioelectrical impedance value R0 at a frequency of 0 Hz and a bioelectrical impedance value Rinf at an infinite frequency which are determined from bioelectrical impedance values measured at a number of frequencies:

$$\alpha = Rinf/R0 .$$

**24.** The apparatus of any one of claims 13 to 16, wherein the parameter $\alpha$ associated with the bioelectrical impedance which represents the intracellular/extracellular fluid ratio is expressed as follows by use of an intracellular fluid resistance value Ri and an extracellular fluid resistance value Re which are calculated based on a bioelectrical impedance value R0 at a frequency of 0 Hz and a bioelectrical impedance value Rinf at an infinite frequency which are determined from bioelectrical impedance values measured at a number of frequencies:

$$\alpha = Ri/Re .$$

## Patentansprüche

**1.** Verfahren zum Schätzen einer Körperzusammensetzung, das Berechnen eines Parameters einer bioelektrischen Impedanz in einem zu messenden Körperteil aus einem Parameterwert eines an einen lebenden Körper anzulegenden Stroms und einem Parameterwert einer gemessenen Spannung umfasst, wobei
eine Körperzusammensetzung geschätzt wird,
**dadurch gekennzeichnet, dass**
ein Parameterwert bioelektrischer Impedanz, der bei einer bestimmten Frequenz gemessen wird, unter Verwendung eines Parameters korrigiert wird, der ein Verhältnis von intrazellulärem zu extrazellulärem Fluid darstellt, und
die Körperzusammensetzung auf Basis des korrigierten Parameterwertes geschätzt wird,
wobei, wenn der mit der bioelektrischen Impedanz zusammenhängende Parameter, der mit dem mit der bioelektrischen Impedanz zusammenhängenden Parameter korrigiert wird, der das Verhältnis von intrazellulärem zu extrazellulärem Fluid darstellt, P' ist, P' entsprechend dem folgenden Korrekturausdruck berechnet wird:

$$P' = f(P,\alpha) = K \cdot P^{A} \cdot \alpha^{B} + C$$

wobei $f(P,\alpha)$ eine Korrekturfunktion ist, die durch Parameter P und $\alpha$ dargestellt wird, P' der mit der bioelektrischen Impedanz zusammenhängende korrigierte Parameter ist, P der mit der bioelektrischen Impedanz zusammenhängende gemessene Parameter ist, $\alpha$ der mit der bioelektrischen Impedanz zusammenhängende Parameter ist, der das Verhältnis von intrazellulärem zu extrazellulärem Fluid darstellt, und A, B, C und K Konstanten sind.

**2.** Verfahren nach Anspruch 1, wobei die bestimmte Frequenz die Frequenz des elektrischen Stroms ist, der zum Schätzen der Körperzusammensetzung an den lebenden Körper angelegt wird.

**3.** Verfahren nach Anspruch 1, wobei die bestimmte Frequenz eine Frequenz ist, die sich von der Frequenz des elektrischen Stroms unterscheidet, der an den lebenden Körper zur Schätzung der Körperzusammensetzung angelegt wird.

**4.** Verfahren nach Anspruch 1, wobei der zu korrigierende Parameter der bioelektrischen Impedanz der Absolutwert der bioelektrischen Impedanz, ein Wert des Vektors der bioelektrischen Impedanz oder der Widerstandskomponenten-Wert des Vektors der bioelektrischen Impedanz ist.

**5.** Verfahren nach einem der Ansprüche 1 bis 4, wobei der mit der bioelektrischen Impedanz zusammenhängende Parameter $\alpha$, der das Verhältnis von intrazellulärem zu extrazellulärem Fluid darstellt, unter Verwendung einer Phasendifferenz $\Phi$ zwischen der Wellenform des an den lebenden Körper angelegten Wechselstroms und der Wellenform der gemessenen Spannung zum Zeitpunkt der Messung der bioelektrischen Impedanz wie folgt ausgedrückt wird:

$$\alpha = 1/\Phi.$$

**6.** Verfahren nach einem der Ansprüche 1 bis 4, wobei der mit der bioelektrischen Impedanz zusammenhängende Parameter $\alpha$, der das Verhältnis von intrazellulärem zu extrazellulärem Fluid darstellt, unter Verwendung einer Phasendifferenz $\Phi$ zwischen der Wellenform des an den lebenden Körper angelegten Wechselstroms und der Wellenform der gemessenen Spannung zum Zeitpunkt der Messung der bioelektrischen Impedanz wie folgt ausgedrückt wird:

$$\alpha = 1/\tan(\Phi).$$

**7.** Verfahren nach einem der Ansprüche 1 bis 4, wobei der mit der bioelektrischen Impedanz zusammenhängende Parameter $\alpha$, der das Verhältnis von intrazellulärem zu extrazellulärem Fluid darstellt, unter Verwendung eines Parameters, der in dem zu korrigierenden, mit der bioelektrischen Impedanz zusammenhängenden Parameter enthalten ist, oder eines mit einer bioelektrischen Impedanz zusammenhängenden Parameters, der bei einer anderen Frequenz gemessen wird, wie folgt ausgedrückt wird:

$$\alpha = R/X$$

wobei R die Widerstandskomponente der bioelektrischen Impedanz ist und X die Reaktanzkomponente der bioelektrischen Impedanz ist.

**8.** Verfahren nach einem der Ansprüche 1 bis 4, wobei der mit der bioelektrischen Impedanz zusammenhängende Parameter $\alpha$, der das Verhältnis von intrazellulärem zu extrazellulärem Fluid darstellt, unter Verwendung des Absolutwertes der bioelektrischen Impedanz oder des Wertes der Widerstandskomponente der bioelektrischen Impedanz, der ein mit einer bioelektrischen Impedanz zusammenhängender Parameter bei höheren und niedrigeren Frequenzen als einer Messfrequenz für den zu korrigierenden, mit der bioelektrischen Impedanz zusammenhängenden Parameter ist, oder desjenigen von ihnen, der der zu korrigierende, mit der bioelektrischen Impedanz zusammenhängende Parameter ist, wie folgt ausgedrückt wird:

$$\alpha = P_{_high}/P_{_low}$$

wobei $P_{_high}$ ein mit einer bioelektrischen Impedanz zusammenhängender Parameter bei einer höheren Frequenz ist und $P_{_low}$ ein mit einer bioelektrischen Impedanz zusammenhängender Parameter bei einer niedrigeren Frequenz ist.

**9.** Verfahren nach einem der Ansprüche 1 bis 4, wobei der mit der bioelektrischen Impedanz zusammenhängende Parameter $\alpha$, der das Verhältnis von intrazellulärem zu extrazellulärem Fluid darstellt, unter Verwendung des Absolutwertes der bioelektrischen Impedanz oder des Wertes der Widerstandskomponente der bioelektrischen Impedanz, der ein mit einer bioelektrischen Impedanz zusammenhängender Parameter bei höheren und niedrigeren Frequenzen als einer Messfrequenz für den zu korrigierenden, mit der bioelektrischen Impedanz zusammenhängenden Parameter ist, oder desjenigen von ihnen, der der zu korrigierende, mit der bioelektrischen Impedanz zusammenhängende Parameter ist, wie folgt ausgedrückt wird:

$$\alpha = P_{_low}/ (P_{_low}-P_{_high})$$

wobei $P_{_high}$ ein mit einer bioelektrischen Impedanz zusammenhängender Parameter bei einer höheren Frequenz ist, und $P_{_low}$ ein mit einer bioelektrischen Impedanz bei einer niedrigeren Frequenz zusammenhängender Parameter ist.

**10.** Verfahren nach einem der Ansprüche 1 bis 4, wobei der mit der bioelektrischen Impedanz zusammenhängende Parameter $\alpha$, der das Verhältnis von intrazellulärem zu extrazellulärem Fluid darstellt, unter Verwendung des Absolutwertes der bioelektrischen Impedanz oder des Wertes der Widerstandskomponente der bioelektrischen Impedanz, der ein mit einer bioelektrischen Impedanz zusammenhängender Parameter bei höheren und niedrigeren Frequenzen als einer Messfrequenz für den zu korrigierenden, mit der bioelektrischen Impedanz zusammenhängenden Parameter ist, oder desjenigen von ihnen, der der zu korrigierende, mit der bioelektrischen Impedanz zusammenhängende Parameter ist, wie folgt ausgedrückt wird:

$$\alpha = P_{_high}/(P_{_low}-P_{_high})$$

wobei $P_{_high}$ ein mit einer bioelektrischen Impedanz zusammenhängender Parameter bei einer höheren Frequenz ist, und $P_{_low}$ ein mit einer bioelektrischen Impedanz bei einer niedrigeren Frequenz zusammenhängender Parameter ist.

**11.** Verfahren nach einem der Ansprüche 1 bis 4, wobei der mit der bioelektrischen Impedanz zusammenhängende Parameter $\alpha$, der das Verhältnis von intrazellulärem Fluid zu extrazellulärem Fluid darstellt, mittels eines Wertes R0 der bioelektrischen Impedanz bei einer Frequenz von 0 Hz und eines Wertes Rinf der bioelektrischen Impedanz bei einer infiniten Frequenz, die anhand von Werten der bioelektrischen Impedanz bestimmt werden, die bei einer Anzahl von Frequenzen gemessen werden, wie folgt ausgedrückt wird:

$$\alpha = Rinf/R0.$$

**12.** Verfahren nach einem der Ansprüche 1 bis 4, wobei der mit der bioelektrischen Impedanz zusammenhängende Parameter $\alpha$, der das Verhältnis von intrazellulärem Fluid zu extrazellulärem Fluid darstellt, unter Verwendung eines Widerstandswertes Ri des intrazellulären Fluids und eines Widerstandswertes Re des extrazellulärem Fluids, die auf Basis eines Wertes R0 der bioelektrischen Impedanz bei 0 Hz und eines Wertes Rinf der bioelektrischen Impedanz bei einer infiniten Frequenz, die anhand von Werten der bioelektrischen Impedanz bestimmt werden, die bei einer Anzahl von Frequenzen gemessen werden, wie folgt ausgedrückt wird:

$$\alpha = Ri/Re.$$

**13.** Vorrichtung (1) zum Messen einer Körperzusammensetzung, die umfasst:

eine Einheit (21) zum Anlegen von elektrischem Strom,
eine Spannungsmesseinheit (22),
eine Einheit (23) zum Berechnen bioelektrischer Impedanz, eine Korrektureinheit (23), und
eine Einheit (23) zum Berechnen einer Körperzusammensetzung,
wobei
die Einheit (21) zum Anlegen von elektrischem Strom einen elektrischen Strom an einen lebenden Körper anlegt,
die Spannungsmesseinheit (22) eine Spannung misst,
die Einheit (23) zum Berechnen bioelektrischer Impedanz einen mit einer bioelektrischen Impedanz eines gemessenen Körperteils zusammenhängenden Parameter anhand des angelegten elektrischen Stroms und der gemessenen Spannung berechnet,
**dadurch gekennzeichnet, dass**
die Korrektureinheit (23) den mit der bei einer bestimmten Frequenz gemessenen bioelektrischen Impedanz zusammenhängenden Parameterwert unter Verwendung eines Parameters korrigiert, der ein Verhältnis von intrazellulärem Fluid zu extrazellulärem Fluid darstellt, und
die Einheit (23) zum Berechnen der Körperzusammensetzung einen mit einer Körperzusammensetzung zusammenhängenden Index auf Basis des mit der bioelektrischen Impedanz zusammenhängenden, korrigierten Parameterwertes berechnet,
wobei, wenn der mit der bioelektrischen Impedanz zusammenhängende Parameter, der mit dem mit der bioelektrischen Impedanz zusammenhängenden Parameter korrigiert worden ist, der das Verhältnis von intrazellulärem Fluid zu extrazellulärem Fluid darstellt, P' ist, die Korrektur des mit der bioelektrischen Impedanz zusammenhängenden Parameters in der Korrektureinheit (23) entsprechend dem folgenden Korrekturausdruck erfolgt:

$$P' = f(P,\alpha) = K \cdot P^A \cdot \alpha^B + C$$

wobei f(P,α) eine Korrekturfunktion ist, die durch Parameter P und α dargestellt wird, P' der mit der bioelektrischen Impedanz zusammenhängende korrigierte Parameter ist, P der mit der bioelektrischen Impedanz zusammenhängende gemessene Parameter ist, α der mit der bioelektrischen Impedanz zusammenhängende Parameter ist, der das Verhältnis von intrazellulärem zu extrazellulärem Fluid darstellt, und A, B, C und K Konstanten sind.

**14.** Vorrichtung nach Anspruch 13, wobei die bestimmte Frequenz die Frequenz des elektrischen Stroms ist, der zum Schätzen der Körperzusammensetzung an den lebenden Körper angelegt wird.

**15.** Vorrichtung nach Anspruch 13, wobei die bestimmte Frequenz eine Frequenz ist, die sich von der Frequenz des elektrischen Stroms unterscheidet, der an den lebenden Körper zur Schätzung der Körperzusammensetzung angelegt wird.

**16.** Vorrichtung nach Anspruch 13, wobei der durch die Korrektureinheit korrigierte Parameter der bioelektrischen Impedanz der Absolutwert der bioelektrischen Impedanz, ein Wert des Vektors der bioelektrischen Impedanz oder der Widerstandskomponenten-Wert des Vektors der bioelektrischen Impedanz ist.

**17.** Vorrichtung nach einem der Ansprüche 13 bis 16, wobei der mit der bioelektrischen Impedanz zusammenhängende Parameter α, der das Verhältnis von intrazellulärem zu extrazellulärem Fluid darstellt, unter Verwendung einer Phasendiflerenz Φ zwischen der Wellenform des an den lebenden Körper angelegten Wechselstroms und der Wellenform der gemessenen Spannung zum Zeitpunkt der Messung der bioelektrischen Impedanz wie folgt ausgedrückt wird:

$$\alpha = 1/\Phi.$$

**18.** Vorrichtung nach einem der Ansprüche 13 bis 16, wobei der mit der bioelektrischen Impedanz zusammenhängende Parameter $\alpha$, der das Verhältnis von intrazellulärem zu extrazellulärem Fluid darstellt, unter Verwendung einer Phasendifferenz $\Phi$ zwischen der Wellenform des an den lebenden Körper angelegten Wechselstroms und der Wellenform der gemessenen Spannung zum Zeitpunkt der Messung der bioelektrischen Impedanz wie folgt ausgedrückt wird:

$$\alpha = 1/\tan(\Phi).$$

**19.** Vorrichtung nach einem der Ansprüche 13 bis 16, wobei der mit der bioelektrischen Impedanz zusammenhängende Parameter $\alpha$, der das Verhältnis von intrazellulärem zu extrazellulärem Fluid darstellt, unter Verwendung eines Parameters, der in dem zu korrigierenden, mit der bioelektrischen Impedanz zusammenhängenden Parameter enthalten ist, oder eines mit einer bioelektrischen Impedanz zusammenhängenden Parameters, der bei einer anderen Frequenz gemessen wird, wie folgt ausgedrückt wird:

$$\alpha = R/X$$

wobei R die Widerstandskomponente der bioelektrischen Impedanz ist und X die Reaktanzkomponente der bioelektrischen Impedanz ist.

**20.** Vorrichtung nach einem der Ansprüche 13 bis 16, wobei der mit der bioelektrischen Impedanz zusammenhängende Parameter $\alpha$, der das Verhältnis von intrazellulärem zu extrazellulärem Fluid darstellt, unter Verwendung des Absolutwertes der bioelektrischen Impedanz oder des Wertes der Widerstandskomponente der bioelektrischen Impedanz, der ein mit einer bioelektrischen Impedanz zusammenhängender Parameter bei höheren und niedrigeren Frequenzen als einer Messfrequenz für den zu korrigierenden, mit der bioelektrischen Impedanz zusammenhängenden Parameter ist, oder desjenigen von ihnen, der der zu korrigierende, mit der bioelektrischen Impedanz zusammenhängende Parameter ist, wie folgt ausgedrückt wird:

$$\alpha = P_{_high}/P_{_low}$$

wobei $P_{_high}$ ein mit einer bioelektrischen Impedanz zusammenhängender Parameter bei einer höheren Frequenz ist und $P_{_low}$ ein mit einer bioelektrischen Impedanz zusammenhängender Parameter bei einer niedrigeren Frequenz ist

**21.** Vorrichtung nach einem der Ansprüche 13 bis 16, wobei der mit der bioelektrischen Impedanz zusammenhängende Parameter $\alpha$, der das Verhältnis von intrazellulärem zu extrazellulärem Fluid darstellt, unter Verwendung des Absolutwertes der bioelektrischen Impedanz oder des Wertes der Widerstandskomponente der bioelektrischen Impedanz, der ein mit einer bioelektrischen Impedanz zusammenhängender Parameter bei höheren und niedrigeren Frequenzen als einer Messfrequenz für den zu korrigierenden, mit der bioelektrischen Impedanz zusammenhängenden Parameter ist, oder desjenigen von ihnen, der der zu korrigierende, mit der bioelektrischen Impedanz zusammenhängende Parameter ist, wie folgt ausgedrückt wird:

$$\alpha = P_{_low}/ (P_{_low}-P_{_high})$$

wobei $P_{_high}$ ein mit einer bioelektrischen Impedanz zusammenhängender Parameter bei einer höheren Frequenz ist, und $P_{_low}$ ein mit einer bioelektrischen Impedanz bei einer niedrigeren Frequenz zusammenhängender Parameter ist.

**22.** Vorrichtung nach einem der Ansprüche 13 bis 16, wobei der mit der bioelektrischen Impedanz zusammenhängende Parameter $\alpha$, der das Verhältnis von intrazellulärem zu extrazellulärem Fluid darstellt, unter Verwendung des Absolutwertes der bioelektrischen Impedanz oder des Wertes der Widerstandskomponente der bioelektrischen Impedanz, der ein mit einer bioelektrischen Impedanz zusammenhängender Parameter bei höheren und niedrigeren Frequenzen als einer Messfrequenz für den zu korrigierenden, mit der bioelektrischen Impedanz zusammenhängenden Parameter ist, oder desjenigen von ihnen, der der zu korrigierende, mit der bioelektrischen Impedanz zusammenhängende Parameter ist, wie folgt ausgedrückt wird:

$$\alpha = P_{_high}/(P_{_low}-P_{_high})$$

wobei $P_{_high}$ ein mit einer bioelektrischen Impedanz zusammenhängender Parameter bei einer höheren Frequenz ist, und $P_{_low}$ ein mit einer bioelektrischen Impedanz bei einer niedrigeren Frequenz zusammenhängender Parameter ist.

**23.** Vorrichtung nach einem der Ansprüche 13 bis 16, wobei der mit der bioelektrischen Impedanz zusammenhängende Parameter $\alpha$, der das Verhältnis von intrazellulärem Fluid zu extrazellulärem Fluid darstellt, mittels eines Wertes R0 der bioelektrischen Impedanz bei einer Frequenz von 0 Hz und eines Wertes Rinf der bioelektrischen Impedanz bei einer infiniten Frequenz, die anhand von Werten der bioelektrischen Impedanz bestimmt werden, die bei einer Anzahl von Frequenzen gemessen werden, wie folgt ausgedrückt wird:

$$\alpha = Rinf/R0.$$

**24.** Vorrichtung nach einem der Ansprüche 13 bis 16, wobei der mit der bioelektrischen Impedanz zusammenhängende Parameter $\alpha$, der das Verhältnis von intrazellulärem Fluid zu extrazellulärem Fluid darstellt, unter Verwendung eines Widerstandswertes Ri des intrazellulären Fluids und eines Widerstandswertes Re des extrazellulärem Fluids, die auf Basis eines Wertes R0 der bioelektrischen Impedanz bei 0 Hz und eines Wertes Rinf der bioelektrischen Impedanz bei einer infiniten Frequenz, die anhand von Werten der bioelektrischen Impedanz bestimmt werden, die bei einer Anzahl von Frequenzen gemessen werden, wie folgt ausgedrückt wird:

$$\alpha = Ri/Re.$$

## Revendications

**1.** Procédé d'estimation de composition corporelle comprenant le calcul d'un paramètre d'une impédance bioélectrique dans une partie de corps à mesurer, à partir d'une valeur de paramètre d'un courant électrique à appliquer à un corps vivant et d'une valeur de paramètre d'une tension mesurée,
dans lequel on estime la composition corporelle,
**caractérisé :**
**en ce qu**'une valeur de paramètre d'une impédance bioélectrique mesurée à une fréquence donnée est corrigée en utilisant un paramètre représentant un rapport de fluide intracellulaire/extracellulaire ; et
en ce que la composition corporelle est estimée en se basant sur la valeur de paramètre corrigée,
dans lequel, lorsque le paramètre associé à l'impédance bioélectrique qui est corrigé par le paramètre associé à l'impédance bioélectrique qui représente le rapport de fluide intracellulaire/extracellulaire est P', on calcule P' selon l'expression de correction suivante :

$$P' = f(P,\alpha) = K \cdot P^A \cdot \alpha^B + C$$

dans laquelle $f(P,\alpha)$ est une fonction de correction représentée par les paramètres P et $\alpha$, P' est le paramètre corrigé associé à l'impédance bioélectrique, P est le paramètre mesuré associé à l'impédance bioélectrique, $\alpha$ est le paramètre associé à l'impédance bioélectrique qui représente le rapport de fluide intracellulaire/extracellulaire, et A, B, C et K sont des constantes.

**2.** Procédé selon la revendication 1, dans lequel la fréquence donnée est la fréquence du courant électrique appliqué au corps vivant pour estimation de la composition corporelle.

**3.** Procédé selon la revendication 1, dans lequel la fréquence donnée est une fréquence différente de la fréquence du courant électrique appliqué au corps vivant pour estimation de la composition corporelle.

**4.** Procédé selon la revendication 1, dans lequel le paramètre à corriger de l'impédance bioélectrique est l'une quelconque de la valeur absolue de l'impédance bioélectrique, d'une valeur vectorielle d'impédance bioélectrique ou de la valeur de composante résistance du vecteur impédance bioélectrique.

**5.** Procédé selon l'une quelconque des revendications 1 à 4, dans lequel le paramètre $\alpha$ associé à l'impédance bioélectrique qui représente le rapport de fluide intracellulaire/extracellulaire s'exprime comme suit par l'utilisation d'une différence de phase $\Phi$ entre la forme d'onde du courant alternatif appliqué au corps vivant et la forme d'onde de la tension mesurée au moment de la mesure de l'impédance bioélectrique :

$$\alpha = 1/\Phi.$$

**6.** Procédé selon l'une quelconque des revendications 1 à 4, dans lequel le paramètre $\alpha$ associé à l'impédance bioélectrique qui représente le rapport de fluide intracellulaire/extracellulaire s'exprime comme suit par l'utilisation d'une différence de phase $\Phi$ entre la forme d'onde du courant alternatif appliqué au corps vivant et la forme d'onde de la tension mesurée au moment de la mesure de l'impédance bioélectrique :

$$\alpha = 1/\tan(\Phi).$$

**7.** Procédé selon l'une quelconque des revendications 1 à 4, dans lequel le paramètre alpha associé à l'impédance bioélectrique qui représente le rapport de fluide intracellulaire/extracellulaire s'exprime comme suit par l'utilisation d'un paramètre inclus dans le paramètre associé à l'impédance bioélectrique à corriger ou d'un paramètre associé à l'impédance bioélectrique qui est mesurée à une autre fréquence :

$$\alpha = R/X$$

où R est la composante résistance de l'impédance bioélectrique, et X est la composante réactance de l'impédance bioélectrique.

**8.** Procédé selon l'une quelconque des revendications 1 à 4, dans lequel le paramètre $\alpha$ associé à l'impédance bioélectrique qui représente le rapport de fluide intracellulaire/extracellulaire s'exprime comme suit par l'utilisation de la valeur absolue de l'impédance bioélectrique ou de la valeur de composante résistance de l'impédance bioélectrique qui est un paramètre associé à une impédance bioélectrique à des fréquences plus haute et plus basse qu'une fréquence de mesure pour le paramètre associé à l'impédance bioélectrique à corriger ou dont l'une ou l'autre est le paramètre associé à l'impédance bioélectrique à corriger :

$$\alpha = P_{_high}/P_{_low}$$

où $P_{_high}$ est un paramètre associé à une impédance bioélectrique à une fréquence plus haute, et $P_{_low}$ est un paramètre associé à une impédance bioélectrique à une fréquence plus basse.

**9.** Procédé selon l'une quelconque des revendications 1 à 4, dans lequel le paramètre $\alpha$ associé à l'impédance bioélectrique qui représente le rapport de fluide intracellulaire/extracellulaire s'exprime comme suit par l'utilisation de la valeur absolue de l'impédance bioélectrique ou de la valeur de composante résistance de l'impédance bioélectrique qui est un paramètre associé à une impédance bioélectrique à des fréquences plus haute et plus basse qu'une fréquence de mesure pour le paramètre associé à l'impédance bioélectrique à corriger ou dont l'une ou l'autre est le paramètre associé à l'impédance bioélectrique à corriger :

$$\alpha = P_{_low}/(P_{_low} - P_{_high})$$

où $P_{_high}$ est un paramètre associé à une impédance bioélectrique à une fréquence plus haute, et $P_{_low}$ est un paramètre associé à une impédance bioélectrique à une fréquence plus basse.

**10.** Procédé selon l'une quelconque des revendications 1 à 4, dans lequel le paramètre $\alpha$ associé à l'impédance bioélectrique qui représente le rapport de fluide intracellulaire/extracellulaire s'exprime comme suit par l'utilisation de la valeur absolue de l'impédance bioélectrique ou de la valeur de composante résistance de l'impédance bioélectrique qui est un paramètre associé à une impédance bioélectrique à des fréquences plus haute et plus basse qu'une fréquence de mesure pour le paramètre associé à l'impédance bioélectrique à corriger ou dont l'une ou l'autre est le paramètre associé à l'impédance bioélectrique à corriger :

$$\alpha = P_{_high}/(P_{_low} - P_{_high})$$

où $P_{_high}$ est un paramètre associé à une impédance bioélectrique à une fréquence plus haute, et $P_{_low}$ est un paramètre associé à une impédance bioélectrique à une fréquence plus basse.

**11.** Procédé selon l'une quelconque des revendications 1 à 4, dans lequel le paramètre $\alpha$ associé à l'impédance bioélectrique qui représente le rapport de fluide intracellulaire/extracellulaire s'exprime comme suit par une valeur R0 d'impédance bioélectrique à une fréquence de 0 Hz et une valeur Rinf d'impédance bioélectrique à une fréquence infinie qui sont déterminées à partir de valeurs d'impédance bioélectrique mesurées à un certain nombre de fréquences :

$$\alpha = Rinf/R0.$$

**12.** Procédé selon l'une quelconque des revendications 1 à 4, dans lequel le paramètre $\alpha$ associé à l'impédance bioélectrique qui représente le rapport de fluide intracellulaire/extracellulaire s'exprime comme suit par l'utilisation d'une valeur Ri de résistance de fluide intracellulaire et d'une valeur Re de résistance de fluide extracellulaire qui sont calculées en se basant sur une valeur R0 d'impédance bioélectrique à une fréquence de 0 Hz et sur une valeur Rinf d'impédance bioélectrique à une fréquence infinie qui sont déterminées à partir de valeurs d'impédance bioélectrique mesurées à un certain nombre de fréquences :

$$\alpha = Ri/Re.$$

**13.** Appareil (1) de mesure de composition corporelle comprenant :

une unité (21) d'application de courant électrique ;
une unité (22) de mesure de tension ;
une unité (23) de calcul d'impédance bioélectrique ;
une unité (23) de correction ; et
une unité (23) de calcul de composition corporelle,
dans lequel l'unité (21) d'application de courant électrique applique un courant électrique à un corps vivant,
dans lequel l'unité (22) de mesure de tension mesure une tension,
dans lequel l'unité (23) d'impédance bioélectrique calcule, à partir du courant électrique appliqué et de la tension mesurée, un paramètre associé à une impédance bioélectrique d'une partie de corps mesurée, **caractérisé :**
**en ce que** l'unité (23) de correction corrige la valeur de paramètre associée à l'impédance bioélectrique mesurée à une fréquence donnée par l'utilisation d'un paramètre représentant un rapport de fluide intracellulaire/extracellulaire ; et
**en ce que** l'unité (23) de calcul de composition corporelle calcule un indice associé à une composition corporelle en se basant sur la valeur de paramètre corrigée associée à l'impédance bioélectrique,

dans lequel, lorsque le paramètre associé à l'impédance bioélectrique qui a été corrigé par le paramètre associé à l'impédance bioélectrique qui représente le rapport de fluide intracellulaire/extracellulaire est P', la correction du paramètre associé à à l'impédance bioélectrique dans l'unité (23) de correction se fait selon l'expression de correction suivante :

$$P' = f(P,\alpha) = K \cdot P^A \cdot \alpha^B + C$$

dans laquelle f(P,α) est une fonction de correction représentée par les paramètres P et α, P' est le paramètre corrigé associé à l'impédance bioélectrique, P est le paramètre mesuré associé à l'impédance bioélectrique, α est le paramètre associé à l'impédance bioélectrique qui représente le rapport de fluide intracellulaire/extracellulaire, et A, B, C et K sont des constantes.

**14.** Appareil selon la revendication 13, dans lequel la fréquence donnée est la fréquence du courant électrique appliqué au corps vivant pour estimation de la composition corporelle.

**15.** Appareil selon la revendication 13, dans lequel la fréquence donnée est une fréquence différente de la fréquence du courant électrique appliqué au corps vivant pour estimation de la composition corporelle.

**16.** Appareil selon la revendication 13, dans lequel le paramètre de l'impédance bioélectrique qui est corrigé par l'unité de correction est l'une quelconque de la valeur absolue de l'impédance bioélectrique, d'une valeur vectorielle d'impédance bioélectrique ou de la valeur de composante résistance du vecteur impédance bioélectrique.

**17.** Appareil selon l'une quelconque des revendications 13 à 16, dans lequel le paramètre α associé à l'impédance bioélectrique qui représente le rapport de fluide intracellulaire/extracellulaire s'exprime comme suit par l'utilisation d'une différence de phase Φ entre la forme d'onde du courant alternatif appliqué au corps vivant et la forme d'onde de la tension mesurée au moment de la mesure de l'impédance bioélectrique :

$$\alpha = 1/\Phi.$$

**18.** Appareil selon l'une quelconque des revendications 13 à 16, dans lequel le paramètre α associé à l'impédance bioélectrique qui représente le rapport de fluide intracellulaire/extracellulaire s'exprime comme suit par l'utilisation d'une différence de phase Φ entre la forme d'onde du courant alternatif appliqué au corps vivant et la forme d'onde de la tension mesurée au moment de la mesure de l'impédance bioélectrique :

$$\alpha = 1/\tan(\Phi).$$

**19.** Appareil selon l'une quelconque des revendications 13 à 16, dans lequel le paramètre alpha associé à l'impédance bioélectrique qui représente le rapport de fluide intracellulaire/extracellulaire s'exprime comme suit par l'utilisation d'un paramètre inclus dans le paramètre associé à l'impédance bioélectrique à corriger ou d'un paramètre associé à l'impédance bioélectrique qui est mesurée à une autre fréquence :

$$\alpha = R/X$$

où R est la composante résistance de l'impédance bioélectrique, et X est la composante réactance de l'impédance bioélectrique.

**20.** Appareil selon l'une quelconque des revendications 13 à 16, dans lequel le paramètre α associé à l'impédance bioélectrique qui représente le rapport de fluide intracellulaire/extracellulaire s'exprime comme suit par l'utilisation de la valeur absolue de l'impédance bioélectrique ou de la valeur de composante résistance de l'impédance bioélectrique qui est un paramètre associé à une impédance bioélectrique à des fréquences plus haute et plus basse qu'une fréquence de mesure pour le paramètre associé à l'impédance bioélectrique à corriger ou dont l'une ou l'autre est le paramètre associé à l'impédance bioélectrique à corriger :

$$\alpha = P_{_high}/P_{_low}$$

où $P_{_high}$ est un paramètre associé à une impédance bioélectrique à une fréquence plus haute, et $P_{_low}$ est un paramètre associé à une impédance bioélectrique à une fréquence plus basse.

**21.** Appareil selon l'une quelconque des revendications 13 à 16, dans lequel le paramètre α associé à l'impédance bioélectrique qui représente le rapport de fluide intracellulaire/extracellulaire s'exprime comme suit par l'utilisation de la valeur absolue de l'impédance bioélectrique ou de la valeur de composante résistance de l'impédance bioélectrique qui est un paramètre associé à une impédance bioélectrique à des fréquences plus haute et plus basse qu'une fréquence de mesure pour le paramètre associé à l'impédance bioélectrique à corriger ou dont l'une ou l'autre est le paramètre associé à l'impédance bioélectrique à corriger :

$$\alpha = P_{_low}/(P_{_low} - P_{_high})$$

où $P_{_high}$ est un paramètre associé à une impédance bioélectrique à une fréquence plus haute, et $P_{_low}$ est un paramètre associé à une impédance bioélectrique à une fréquence plus basse.

**22.** Appareil selon l'une quelconque des revendications 13 à 16, dans lequel le paramètre α associé à l'impédance bioélectrique qui représente le rapport de fluide intracellulaire/extracellulaire s'exprime comme suit par l'utilisation de la valeur absolue de l'impédance bioélectrique ou de la valeur de composante résistance de l'impédance bioélectrique qui est un paramètre associé à une impédance bioélectrique à des fréquences plus haute et plus basse qu'une fréquence de mesure pour le paramètre associé à l'impédance bioélectrique à corriger ou dont l'une ou l'autre est le paramètre associé à l'impédance bioélectrique à corriger :

$$\alpha = P_{_high}/(P_{_low} - P_{_high})$$

où $P_{_high}$ est un paramètre associé à une impédance bioélectrique à une fréquence plus haute, et $P_{_low}$ est un paramètre associé à une impédance bioélectrique à une fréquence plus basse.

**23.** Appareil selon l'une quelconque des revendications 13 à 16, dans lequel le paramètre α associé à l'impédance bioélectrique qui représente le rapport de fluide intracellulaire/extracellulaire s'exprime comme suit par une valeur R0 d'impédance bioélectrique à une fréquence de 0 Hz et une valeur Rinf d'impédance bioélectrique à une fréquence infinie qui sont déterminées à partir de valeurs d'impédance bioélectrique mesurées à un certain nombre de fréquences :

$$\alpha = Rinf/R0.$$

**24.** Appareil selon l'une quelconque des revendications 13 à 16, dans lequel le paramètre α associé à l'impédance bioélectrique qui représente le rapport de fluide intracellulaire/extracellulaire s'exprime comme suit par l'utilisation d'une valeur Ri de résistance de fluide intracellulaire et d'une valeur Re de résistance de fluide extracellulaire qui sont calculées en se basant sur une valeur R0 d'impédance bioélectrique à une fréquence de 0 Hz et sur une valeur Rinf d'impédance bioélectrique à une fréquence infinie qui sont déterminées à partir de valeurs d'impédance bioélectrique mesurées à un certain nombre de fréquences :

$$\alpha = Ri/Re.$$

FIG. 1

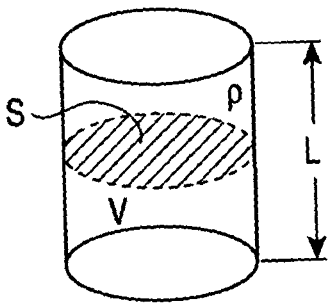


FIG. 2

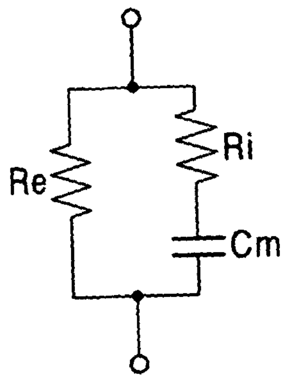


FIG. 3

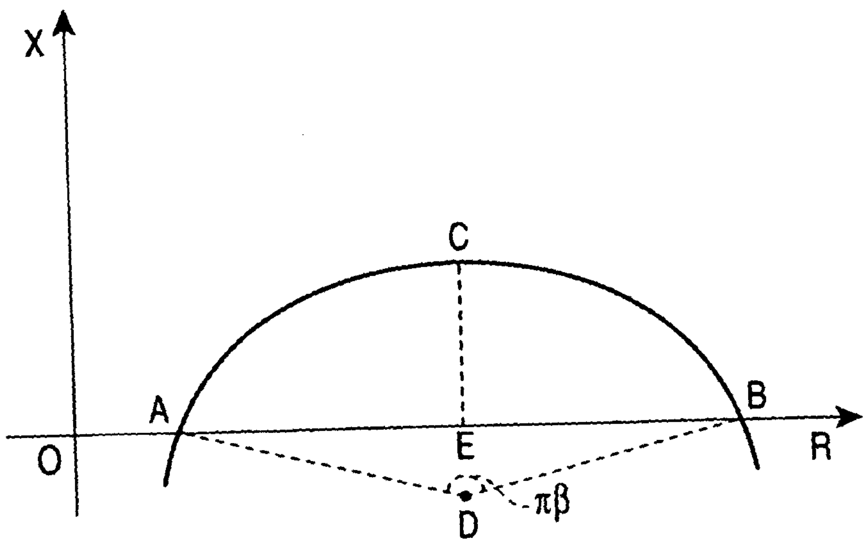

FIG. 4

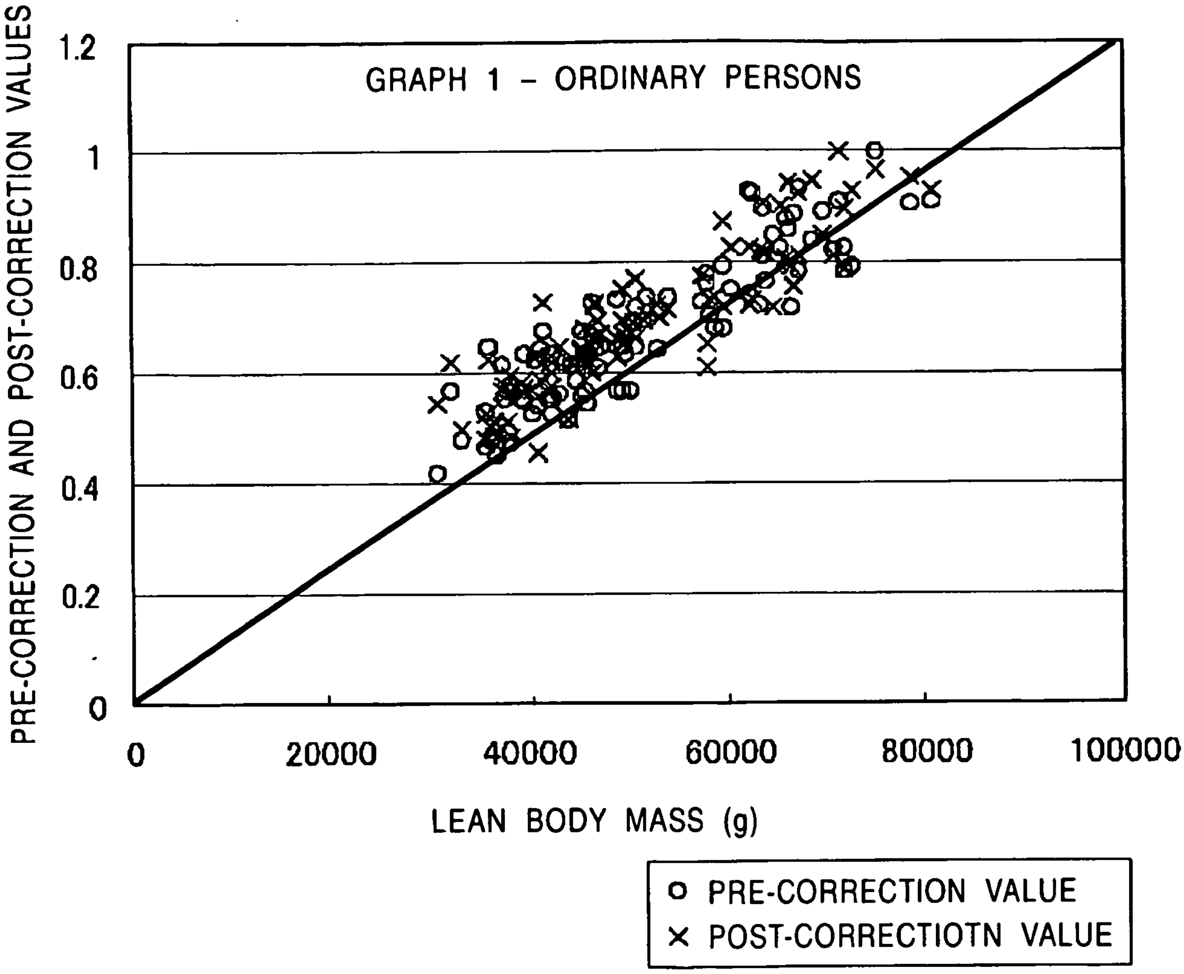
GRAPH 1 - ORDINARY PERSONS
PRE-CORRECTION AND POST-CORRECTION VALUES
1.2
1
0.8
0.6
0.4
0.2
0
0
20000
40000
60000
80000
100000
LEAN BODY MASS (g)
o PRE-CORRECTION VALUE
× POST-CORRECTIOTN VALUE

FIG. 5

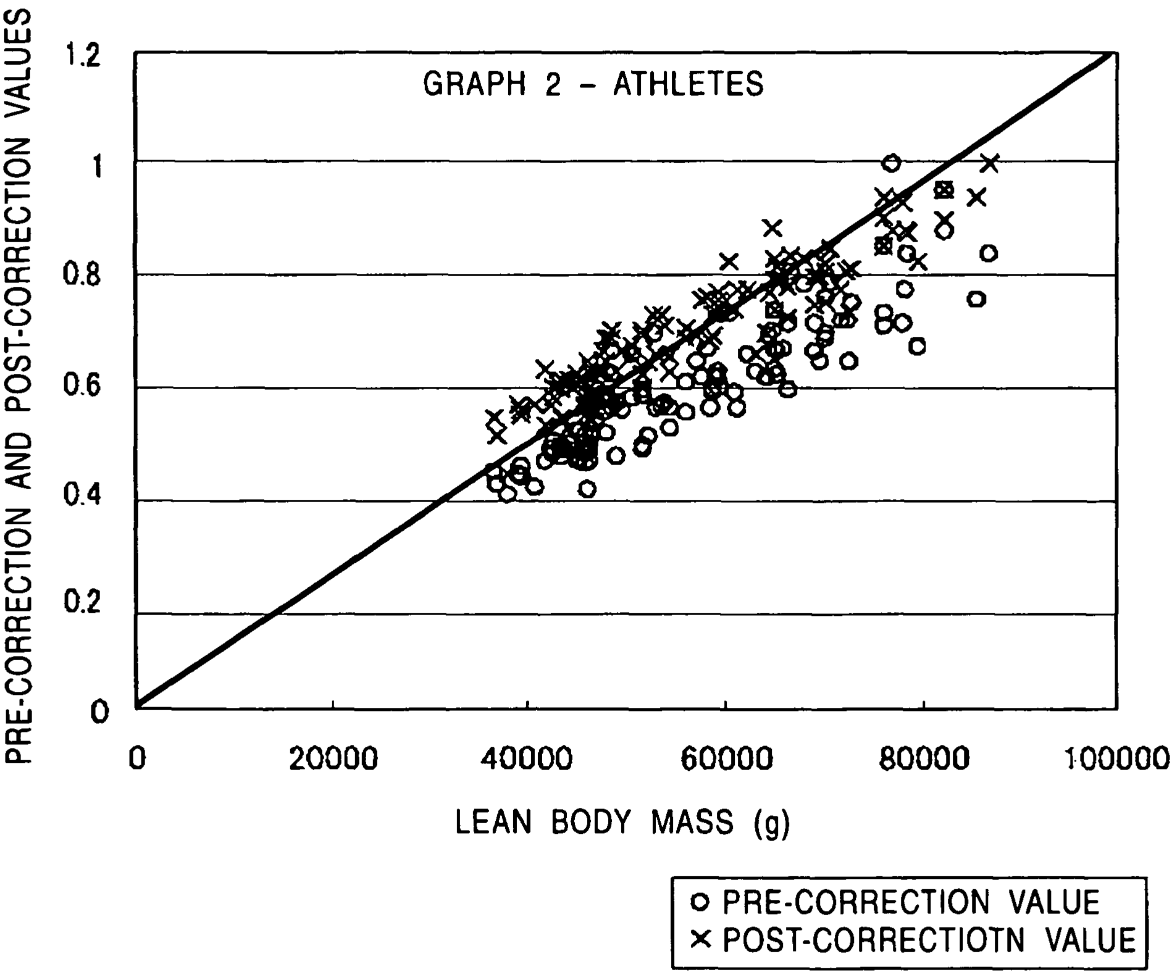
GRAPH 2 - ATHLETES
PRE-CORRECTION AND POST-CORRECTION VALUES
1.2
1
0.8
0.6
0.4
0.2
0
0
20000
40000
60000
80000
100000
LEAN BODY MASS (g)
PRE-CORRECTION VALUE
POST-CORRECTIOTN VALUE

FIG. 6

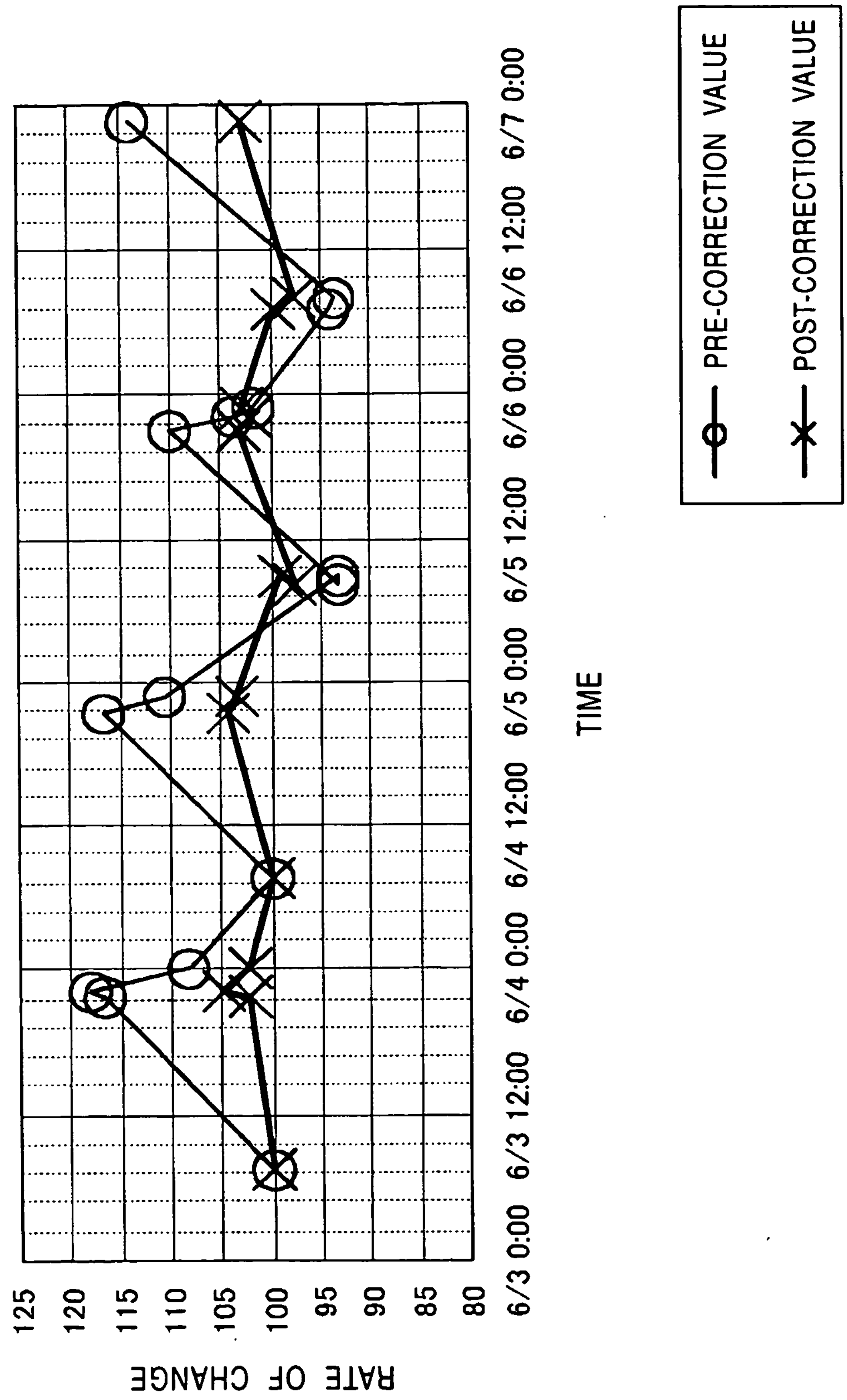
RATE OF CHANGE
125
120
115
110
105
100
95
90
85
80
TIME
6/3 0:00
6/3 12:00
6/4 0:00
6/4 12:00
6/5 0:00
6/5 12:00
6/6 0:00
6/6 12:00
6/7 0:00
PRE-CORRECTION VALUE
POST-CORRECTION VALUE

FIG. 7

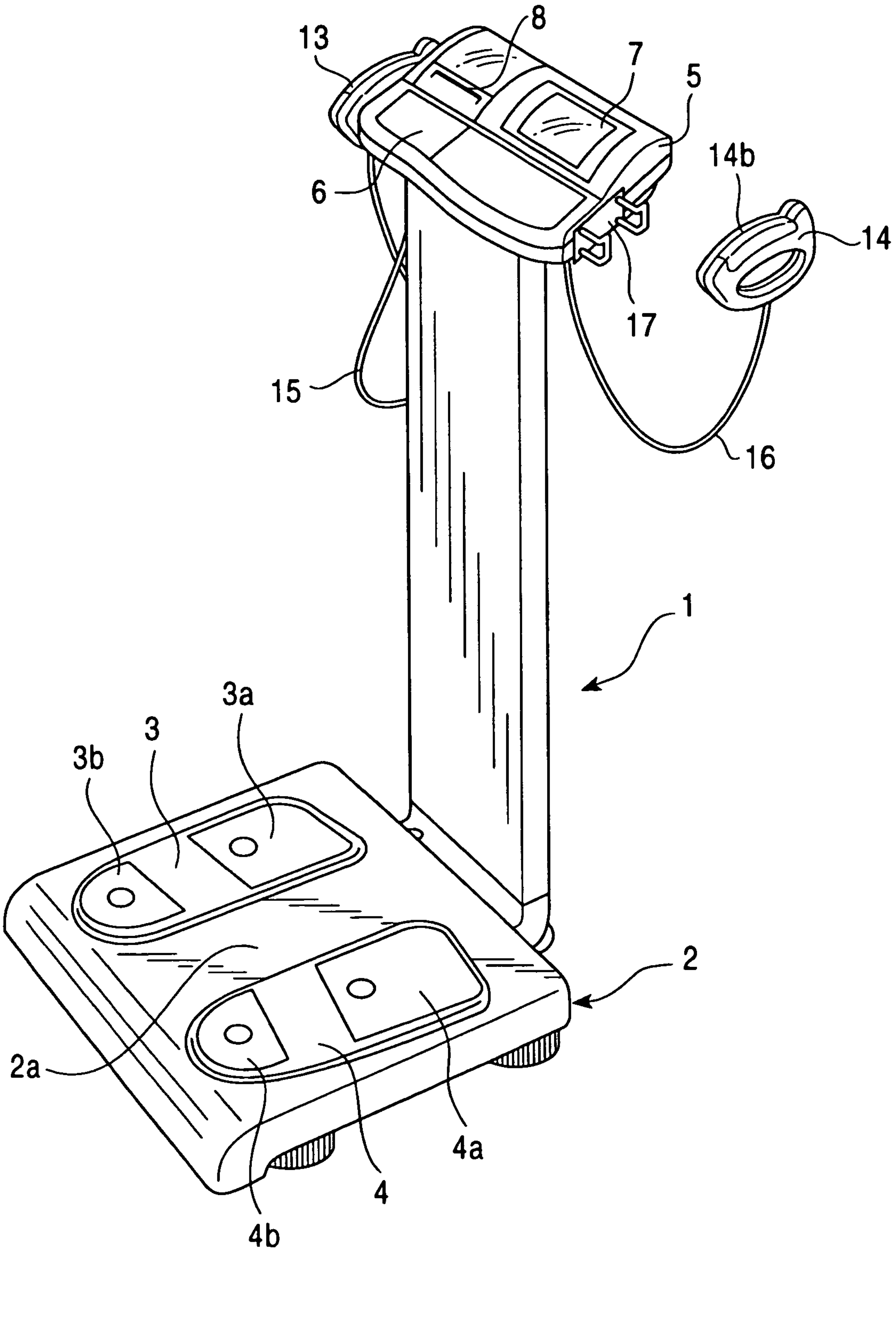

FIG. 8

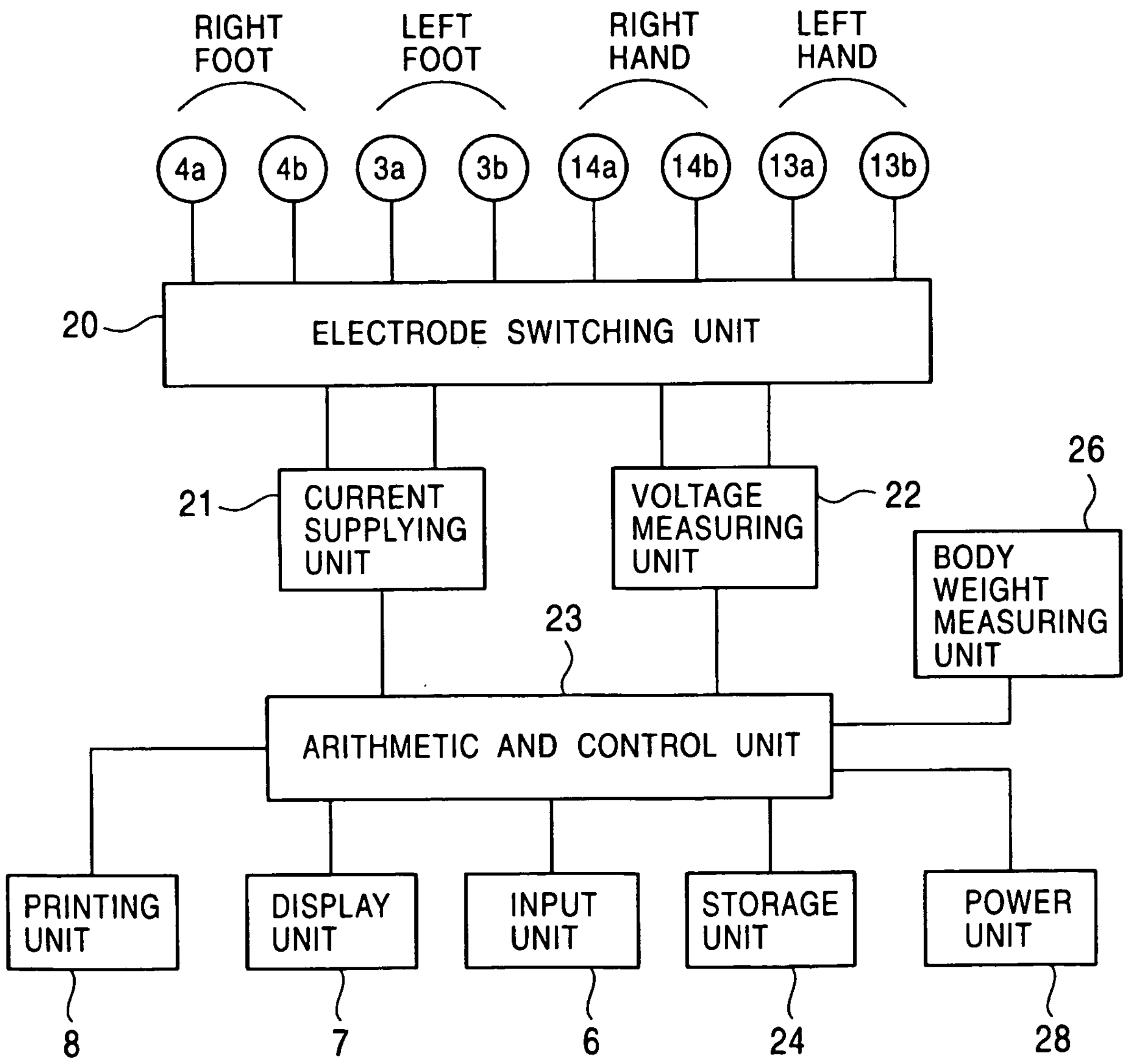
RIGHT FOOT
LEFT FOOT
RIGHT HAND
LEFT HAND
4a
4b
3a
3b
14a
14b
13a
13b
20
ELECTRODE SWITCHING UNIT
21
CURRENT SUPPLYING UNIT
VOLTAGE MEASURING UNIT
22
26
BODY WEIGHT MEASURING UNIT
23
ARITHMETIC AND CONTROL UNIT
PRINTING UNIT
DISPLAY UNIT
INPUT UNIT
STORAGE UNIT
POWER UNIT
8
7
6
24
28

FIG. 9

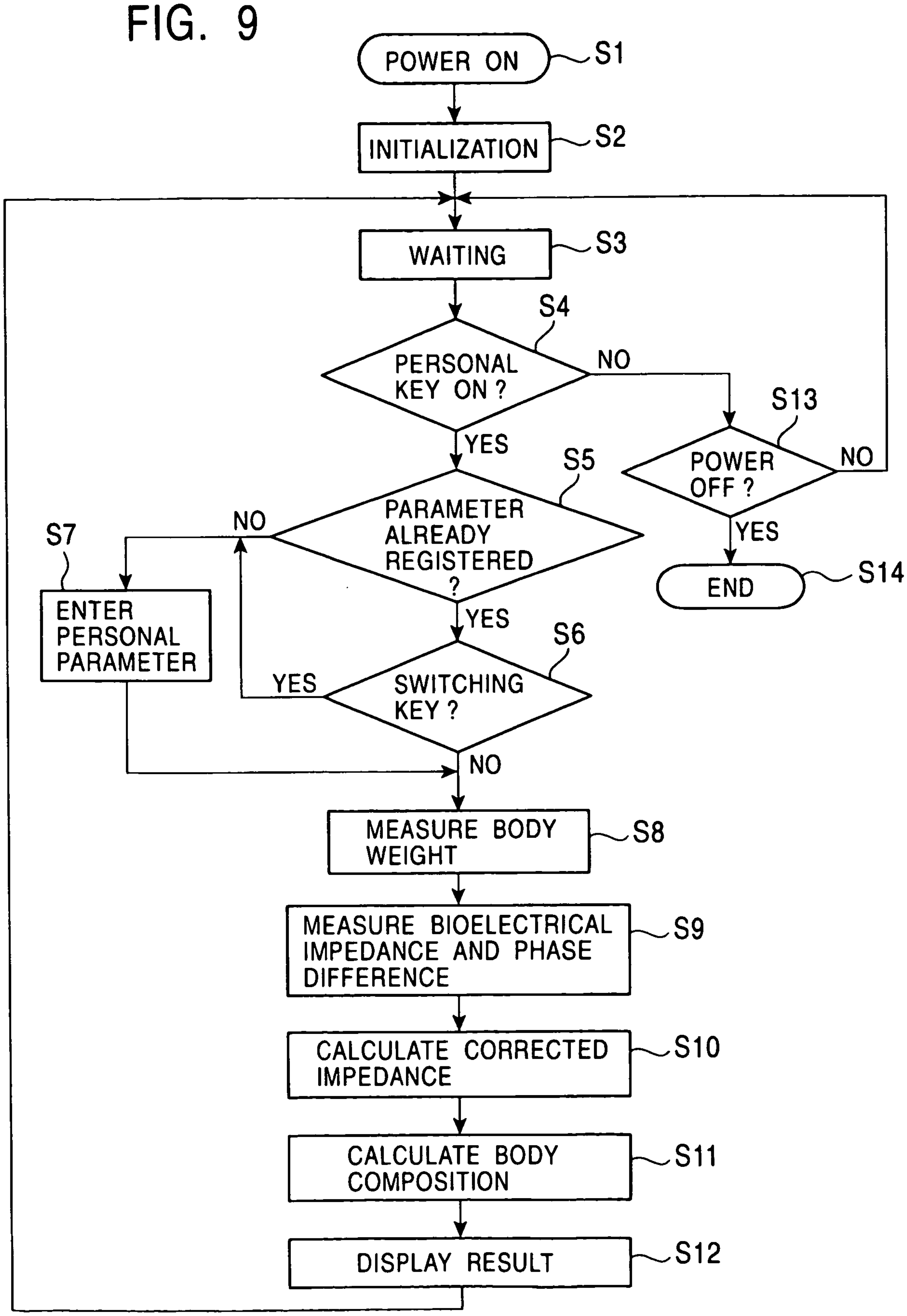
POWER ON
S1
INITIALIZATION
S2
WAITING
S3
S4
PERSONAL KEY ON ?
NO
YES
S13
POWER OFF ?
NO
YES
END
S14
S5
PARAMETER ALREADY REGISTERED ?
NO
YES
S7
ENTER PERSONAL PARAMETER
S6
SWITCHING KEY ?
YES
NO
MEASURE BODY WEIGHT
S8
MEASURE BIOELECTRICAL IMPEDANCE AND PHASE DIFFERENCE
S9
CALCULATE CORRECTED IMPEDANCE
S10
CALCULATE BODY COMPOSITION
S11
DISPLAY RESULT
S12

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 10005187 A
- JP 9051884 A
- JP 3330951 B